# EUROPEAN PATENT APPLICATION

(11) **EP 4 631 490 A1**
(43) Date of publication of application: **15.10.2025**
(21) Application number: 23900605.9
(22) Date of filing: 04.12.2023
(51) Int. Cl.: A61K 8/60, A61K 8/02, A61K 8/34, A61K 8/73, A61K 9/06, A61K 31/7024, A61K 47/10, A61K 47/26, A61K 47/44, A61P 17/00, A61P 43/00, A61Q 1/00, A61Q 19/00, A61Q 19/08

(54) **ACTIVATOR AND SKIN CONDITION IMPROVING AGENT**

(30) Priority: 05.12.2022 JP 2022194276; 01.05.2023 JP 2023075519; 15.05.2023 JP 2023080193
(71) Applicant: Nippon Shokubai Co., Ltd., Osaka-shi, Osaka 541-0043 (JP)
(72) Inventor: HORIKAWA, Hiroshi, Suita-shi, Osaka 564-0034 (JP); OTAKE, Tetsuo, Suita-shi, Osaka 564-0034 (JP); NAKANOSHO, Masahiro, Suita-shi, Osaka 564-0034 (JP); MUSHIGA, Sumito, Suita-shi, Osaka 564-0034 (JP); TAKASE, Hiroyuki, Suita-shi, Osaka 564-0034 (JP)
(74) Representative: Graf von Stosch Patentanwaltsgesellschaft mbH
(86) International application number: PCT/JP2023/043237
(87) International publication number: WO 2024/122490

(57) **Abstract**

An object is to provide a novel activator and a skin condition improving agent. This object is achieved by an activator containing an anionic biosurfactant, the activator containing an anionic biosurfactant, and a skin condition improving agent.

## Description

### Technical Field

The present invention relates to an activator containing an anionic biosurfactant and a skin condition improving agent containing an anionic biosurfactant. Further, the present invention relates to a cosmetic or quasi-drug using the activator.

### Background Art

Human skin is greatly stimulated by ultraviolet rays, air pollution, makeup, and the like. To reduce the stimulation of the skin, external preparations for skin, which are cosmetics or quasi-drugs, contain physiologically active components and generally have effects such as moisture retention, prevention of rough skin, prevention of spots and freckles, skin tightening, anti-inflammation, promotion of blood circulation, and prevention of skin deterioration due to aging. Examples of the physiologically active components include a moisture-retaining component, an emollient component, an astringent component, a keratin softening component, a whitening component, an anti-inflammation (anti-inflammatory) component, an anti-ultraviolet component, an antioxidative component, a cell-activating component, and a blood-circulation-promoting component, and they are blended in the external preparations depending on respective purposes.

As such a cosmetic or quasi-drug containing physiologically active components, for example, Patent Document 1 describes that a composition for external application to the skin containing an extract of Momordica grosvenorii and a sophorolipid has a fibroblast activation action and a collagen synthesis action, and has a rough skin improvement effect and an ultraviolet protection effect.

Patent Document 2 describes that a mannosylerythritol lipid (MEL) inhibits formation of skin melanocytes, improves overall skin tone to make the skin beautiful, and provides a whitening efficacy of improving dark color tone of the skin to make the skin bright.

On the other hand, a composition containing an anionic surfactant has been used as a cleansing composition having excellent skin feeling while having cleansing power. For example, Patent Document 3 describes that a skin cleansing composition containing specific amounts of an anionic surfactant such as acyl isethionic acid and a salt thereof, alkyl sulfate or polyoxyethylene alkyl sulfate, or 2-ethylhexyl glyceryl ether, and water is excellent in foam volume and cleansing power upon dilution, generates many shed cells, provides smooth skin after cleansing, and is excellent in makeup coverage of makeup cosmetics applied onto the skin after cleansing.

### Citation List

### Patent Document

Patent Document 1: JP 2007-106733 A
Patent Document 2: JP 2019-526602 T
Patent Document 3: JP 2015-124214 A

### Summary of Invention

### Technical Problem

Under the circumstances described above, an embodiment of the present invention is directed to providing a novel activator and a novel skin condition improving agent.

### Solution to Problem

As a result of various investigations to achieve the above object, the present inventors have found that an anionic biosurfactant has a good activating ability and is useful as a cell activator, and have completed the present invention. Furthermore, as described in Patent Document 1 that a surfactant in a detergent is an external stress, many anionic surfactants in the related art as described in Patent Document 3 include compounds having skin irritation. In contrast, the present inventors have found that the anionic biosurfactant described above has low skin irritation and good foaming durability and can be suitably used as products to be applied to human skin such as cosmetics and quasi-drugs. In addition, the present inventors have also found that the anionic biosurfactant can be suitably used as various skin condition improving agents. That is, an aspect of the present invention relates to an activator containing an anionic biosurfactant, a skin condition improving agent containing an anionic biosurfactant, and a cosmetic and a quasi-drug containing the activator.

### Advantageous Effects of Invention

According to an embodiment of the present invention, a novel activator and a novel skin condition improving agent can be provided. Thus, for example, it is possible to provide an activator which is useful as a cell activator and has low skin irritation and good foaming durability, various skin condition improving agents, and a cosmetic and a quasi-drug containing the activator.

### Description of Embodiments

### 1. Activator

An activator according to an embodiment of the present invention includes an anionic biosurfactant.

In the present specification, the term "activation" means maintaining or enhancing a cellular function or cellular activity. The term "activator" means a substance that has an activating ability and, as a result, can suppress a decrease in cell function or cell activity. Thus, the "activator" is synonymous with a "cell activator".

In the present specification, the term "anionic biosurfactant" means a biosurfactant having an anionic property. In the present specification, the term "anionic" means having a property that a hydrophilic group is ionized in water to be negatively charged. In order for a compound to be anionic, it is necessary to have an acidic functional group such as a carboxyl group, a sulfonic acid group, or a sulfate ester group. Thus, it can be said that the anionic biosurfactant according to an embodiment of the present invention is a biosurfactant having an acidic functional group. The term "biosurfactant" as used herein is a generic term for substances (compounds) that are produced (biosynthesized) by organisms (e.g., microorganisms) and have surface activating ability and/or emulsifying ability.

The anionic biosurfactant contained in the activator according to an embodiment of the present invention is not particularly limited as long as it is anionic and is a substance produced by a living organism and having surface activating ability and/or emulsifying ability. Examples thereof include a biosurfactant having a glycolipid structure, a biosurfactant having an amino acid structure, a biosurfactant having an organic acid structure, and a biosurfactant having a macromolecular structure. The activator according to an embodiment of the present invention may contain only one kind of the various anionic biosurfactants described above or may contain two or more kinds thereof.

Examples of the biosurfactant having a glycolipid structure that can be contained in the activator according to an embodiment of the present invention include a succinoyl trehalose lipid (hereinafter, also referred to as STL) and a sophorolipid.

The anionic biosurfactant contained in the activator according to an embodiment of the present invention is preferably a biosurfactant produced by a microorganism, more preferably a biosurfactant having a glycolipid structure, and still more preferably a succinoyl trehalose lipid, from the viewpoint of foaming durability and low skin irritation. In other words, the activator according to an embodiment of the present invention preferably contains an anionic biosurfactant containing a biosurfactant produced by a microorganism, more preferably contains an anionic biosurfactant containing a biosurfactant having a glycolipid structure, and still more preferably contains an anionic biosurfactant containing a succinoyl trehalose lipid.

The succinoyl trehalose lipid that can be contained in the activator according to an embodiment of the present invention can be prepared by aerobically culturing Rhodococcus erythropolis strain SD-74 in a medium containing a carbon source such as fatty acid or vegetable oil and/or fat, or by aerobically culturing Rhodococcus sp. strain TB-42 in a medium containing a carbon source such as unsaturated hydrocarbon or halogenated hydrocarbon. Note that the Rhodococcus erythropolis strain SD-74 is deposited as "accession number: NITE BP-03788 (date of deposit: December 1, 2022)" with the National Institute of Technology and Evaluation, Patent Microorganisms Depositary (address: Room #122, 2-5-8 Kazusakamatari, Kisarazu City, Chiba Prefecture, 292-0818).

A succinoyl trehalose lipid prepared by culturing microorganisms in a medium containing a carbon source is a glycolipid in which the saccharide moiety is trehalose, one to two moles of succinic acid are ester-bonded per one mole of trehalose and one to two moles of a fatty acid are ester-bonded per one mole of trehalose. The fatty acid moiety of the glycolipid is derived from the carbon source which is a culture substrate, and different fatty acids can be bonded by changing the composition of the carbon source.

The succinoyl trehalose lipid that can be contained in the activator according to an embodiment of the present invention may be, for example, a compound having a structure of the following general formula 1.

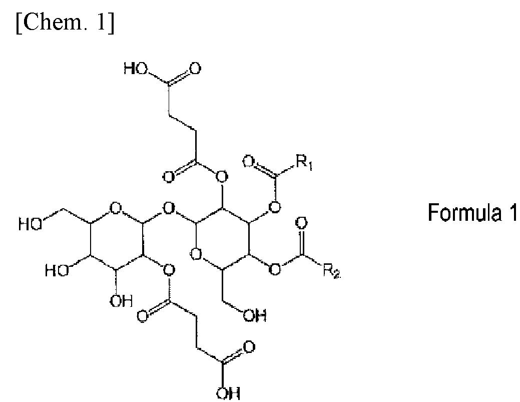

In the general formula 1, the substituents R₁ and R₂ are aliphatic hydrocarbon groups.

In an embodiment of the present invention, the aliphatic hydrocarbon groups which are the substituents R₁ and R₂ in the general formula 1 may each independently be linear or branched, and are preferably linear from the viewpoint of assimilation of the raw material carbon source.

In an embodiment of the present invention, the aliphatic hydrocarbon groups which are the substituents R₁ and R₂ in the general formula 1 may each independently be an alkyl group having 5 to 30 carbon atoms. The alkyl group preferably has 8 or more carbon atoms, more preferably 10 or more carbon atoms, and still more preferably 12 or more carbon atoms. Further, the alkyl group preferably has 25 or less carbon atoms, more preferably 22 or less carbon atoms, and still more preferably 20 or less carbon atoms.

The succinoyl trehalose lipid having the structure of the general formula 1 can be prepared, for example, by aerobically culturing the Rhodococcus erythropolis strain SD-74 in a medium containing a carbon source such as a fatty acid or a vegetable oil and/or fat, and/or by aerobically culturing the Rhodococcus sp. strain TB-42 in a medium containing a carbon source such as unsaturated hydrocarbon or halogenated hydrocarbon.

In a case where the succinoyl trehalose lipid is produced by culturing a microorganism in a medium containing a carbon source containing an alkylene oxide, the resulting succinoyl trehalose lipid may contain a structural unit derived from an alkylene oxide adduct in the substituent R₁ and/or R₂. The succinoyl trehalose lipid according to an embodiment of the present invention preferably has a structure in which the content of the structural unit derived from the alkylene oxide adduct is small from the viewpoint of maintaining the hydrophilic-hydrophobic balance of the entire molecular structure, and specifically, the content of the structural unit derived from the alkylene oxide adduct in the substituents R₁ and R₂ in the general formula 1 is preferably 20 mass% or less, more preferably 10 mass% or less, and still more preferably 5 mass% or less, and may be 0 parts by mass.

The content of the anionic biosurfactant in 100 parts by mass of the activator according to an embodiment of the present invention is preferably 95 parts by mass or more, more preferably 98 parts by mass or more, and still more preferably 99 parts by mass or more, and may be 100 parts by mass from the viewpoint of maintaining high purity and exhibiting stable performance (for example, activating effect).

One of the features of the activator according to an embodiment of the present invention is that the activator has not only an activating effect but also low skin irritation and good foaming durability. Note that the skin irritation and the foaming durability of the activator can be measured and evaluated by the methods described in Examples.

The critical micelle concentration (CMC) of the activator according to an embodiment of the present invention is preferably 1000 × 10⁻⁶ M or less, more preferably 500 × 10⁻⁶ M or less, and still more preferably 200 × 10⁻⁶ M or less, from the viewpoint of surface activating ability when used as a cleansing agent. Note that the critical micelle concentration of the activator can be measured by the method described in Examples.

The activator according to an embodiment of the present invention has a 50% cell growth inhibitory concentration (IC50) of preferably 100 ppm or more, and more preferably 300 ppm or more. A higher IC50 of an activator means that the activator is less cytotoxic. Note that the IC50 of the activator can be measured by the method described in Examples.

The activator according to an embodiment of the present invention may contain a solvent in addition to the anionic biosurfactant. In other words, the activator according to an embodiment of the present invention may be provided in a form of a solution in which the anionic biosurfactant is dissolved in a solvent. The solvent that can be contained in the activator according to an embodiment of the present invention is not particularly limited as long as it can dissolve the anionic biosurfactant according to an embodiment of the present invention, and examples thereof include water, 1,3-butylene glycol, ethanol, acetone, and methanol. Among them, water or 1,3-butylene glycol is preferable from the viewpoint of use as a cosmetic raw material.

In a case where the activator according to an embodiment of the present invention contains a solvent, the content (concentration) of the solvent is not particularly limited, and may be, for example, from 0.1 to 50 wt.% in 100 mass% of the activator according to an embodiment of the present invention.

The activator according to an embodiment of the present invention may contain additional additives besides the anionic biosurfactant and the solvent.

Examples of the additional additives according to an embodiment of the present invention include oils and/or fats, waxes, mineral oils, fatty acids, alcohols, esters, metal soaps, gums and water-soluble macromolecular compounds, vitamins, amino acids, whitening agents, moisturizers, hair growth agents, α-hydroxy acids, inorganic pigments, ultraviolet absorbers, astringents, antioxidants, anti-inflammatory agents, bactericides and disinfectants, flavors, dyes and colorants, hormones, sequestering agents, pH adjusters, chelating agents, antiseptic and antifungal agents, cooling agents, stabilizers, animal and plant proteins and decomposition products thereof, animal and plant polysaccharides and decomposition products thereof, animal and plant glycoproteins and decomposition products thereof, blood flow promoters, anti-inflammatory and antiallergic agents, cell activators, keratolytic agents, wound healing agents, foam boosters, thickeners, oral agents, and refresher and deodorant agents.

### Method for producing activator

Examples of the method for producing an activator according to an embodiment of the present invention include a method including a culture step of culturing a microorganism in a medium containing a carbon source, a precipitation step of precipitating a product prepared by the culture step, an extraction step of extracting an extract containing an anionic biosurfactant (e.g., an STL composition) from the precipitate prepared by the precipitation step, and a fat-soluble substance removing step of removing a fat-soluble substance from the extract prepared by the extraction step. Hereinafter, the production method will be described in detail.

### Culture step

In the culture step, the step of culturing a microorganism in a medium containing a carbon source is performed in accordance with a method in the related art. Nutrients such as a nitrogen source and an inorganic salt may be added to the medium to which the carbon source has been added, as necessary.

The "microorganism" in the culture step is not particularly limited as long as it can generate an anionic biosurfactant, but is preferably a microorganism belonging to the genus Rhodococcus and more preferably a Rhodococcus erythropolis strain SD-74, a Rhodococcus sp. strain TB-42, or a Rhodococcus baikonurensis strain NBRC 100611 because they can produce succinoyl trehalose lipids.

The "carbon source" in the culture step is preferably a carbon compound assimilated by the microorganism during culture, and particularly preferably a natural oil and/or fat, a hydrocarbon, a fatty acid, a fatty acid ester, or a higher alcohol. Here, the carbon compound means a compound of carbon with hydrogen, nitrogen, and the like. The carbon source to be used in the culture step may be, for example, a natural oil and/or fat, and may be an animal oil and/or fat or a vegetable oil and/or fat, but is preferably a vegetable oil and/or fat because it is more easily available. The vegetable oil and/or fat to be used in the culture step is preferably, but not limited to, palm oil, coconut oil, soybean oil, olive oil, safflower oil, rapeseed oil, corn oil, cottonseed oil, tall oil, or the like.

Alternatively, hydrocarbon may be used as a carbon source in the culture step. Suitable examples of the hydrocarbon to be used as the carbon source include normal alkanes such as n-decane, n-undecane, n-tridecane, n-tetradecane, n-pentadecane, n-hexadecane, n-heptadecane, n-octadecane, and n-nonadecane; and normal alkenes such as 1-decene, 1-undecene, 1-dodecene, 1-tridecene, 1-tetradecene, 1-pentadecene, 1-hexadecene, 1-heptadecene, and 1-octadecene.

As the hydrocarbon to be used as the carbon source, n-tridecane, n-tetradecane, n-pentadecane, n-hexadecane, or n-heptadecane is preferable, and n-tetradecane, n-pentadecane, or n-hexadecane is more preferable, from the viewpoint of improving the proliferation property of the microorganism and the productivity of the anionic biosurfactant, and n-tetradecane is particularly preferable because various physiological functions (e.g., skin barrier property, moisture retention property, skin elasticity improving property, inhibition of melanin generation, and the like) of the resulting anionic biosurfactant can be improved.

A fatty acid can also be used as the carbon source in the culture step. Suitable examples of the fatty acid to be used as the carbon source include decanoic acid, undecanoic acid, dodecanoic acid (lauric acid), tridecanoic acid, tetradecanoic acid (myristic acid), pentadecanoic acid, hexadecanoic acid (palmitic acid), heptadecanoic acid, octadecanoic acid (stearic acid), and oleic acid. A fatty acid ester, or a higher alcohol such as undecyl alcohol or dodecyl alcohol (lauryl alcohol) may also be used as the carbon source.

As the fatty acid to be used as the carbon source, tridecanoic acid, tetradecanoic acid (myristic acid), pentadecanoic acid, hexadecanoic acid (palmitic acid) and heptadecanoic acid are preferable, and tetradecanoic acid (myristic acid), pentadecanoic acid, and hexadecanoic acid (palmitic acid) are more preferable, from the viewpoint of improving the proliferation property of the microorganism and the productivity of the anionic biosurfactant, and tetradecanoic acid (myristic acid) is particularly preferable because it can improve various physiological functions (e.g., skin barrier property, moisture retention property, skin elasticity improving property, inhibition of melanin generation, and the like) of the resulting anionic biosurfactant.

As the carbon source to be added to the medium, each carbon source described above is suitably used. The concentration of the carbon source to be added to the medium is preferably from 5 to 20 mass%, and more preferably 10 mass%. In addition to the carbon source, a nitrogen source is preferably added to the medium to be used in the culture step. As the nitrogen source to be added to the medium, a nitrogen-containing organic or inorganic substance to be usually used for culturing microorganisms is used, and for example, sodium nitrate, potassium nitrate, potassium hydrogen phosphate, potassium dihydrogen phosphate, and the like can be used. In addition to the above, a nutrient such as yeast extract or peptone may be added to the medium if necessary for growing the microorganism.

Culture of the microorganism in the culture step is preferably performed under aerobic conditions by shaking and stirring. The culture temperature is preferably from 20 to 35°C, and more preferably 30°C. The culture pH is preferably from 5.5 to 9.5. As the culture period, culture is preferably continued until a mixture containing the anionic biosurfactant (e.g., succinoyl trehalose lipid) at a concentration of 15 to 150 g/L is generated. In Examples described below, the concentration of succinoyl trehalose lipid reaches 25 g/L in 332 hours of culture, and thus the culture is preferably continued for 199 to 664 hours.

In the culture step, seed culture may be performed before the main culture of the microorganism. By seed culture of the microorganism, the microorganism can be adjusted to optimum conditions, and as a result, succinoyl trehalose lipid can be efficiently produced.

### Precipitation step

The precipitation step is a step of precipitating a product containing the anionic biosurfactant (for example, a product containing STL) produced by the microorganism in the culture step. That is, precipitation is performed in the medium or the culture solution containing the anionic biosurfactant generated in the medium by the microorganism. At this time, the medium or the culture solution serving as a target of precipitation may be prepared by centrifuging a culture solution in which microorganism has been cultured to remove bacterial cells and a residual substrate from the culture solution. The term "precipitate" as used herein means that a substance dissolved in a medium is taken out as a solid. That is, in the precipitation step according to an embodiment of the present invention, the anionic biosurfactant such as a glycolipid generated in the medium by the microorganism in the culture step can be taken out as a solid in the medium.

In the precipitation step, a method for precipitating the product containing the anionic biosurfactant is not particularly limited as long as it is a method capable of separating the product containing the anionic biosurfactant as a solid in the culture solution, and a method in the related art can be used. For example, a product containing an acidic anionic biosurfactant can be precipitated by acidifying the medium in which the microorganism has been cultured in the culture step to precipitate an acidic substance in the medium (acid precipitation). Specifically, a product containing an anionic biosurfactant can be precipitated by lowering the pH of the culture solution (target). To lower the pH of the culture solution, an acidic substance such as HCl only need be added. Thereafter, the precipitated product can be taken out by performing, for example, centrifugal treatment. Hereinafter, the product prepared through the precipitation step is referred to as a "precipitation product".

### Extraction step

The extraction step is a step of extracting an STL composition from the precipitation product. In the extraction step, a solvent which is incompatible with water and in which an anionic biosurfactant, particularly a glycolipid, is soluble is added to the precipitation product to dissolve the anionic biosurfactant contained in the precipitation product in a solvent layer. Then, when the solvent layer in which the anionic biosurfactant is dissolved is separated, an anionic biosurfactant composition from which a water-soluble substance has been removed can be prepared. Note that the "water-soluble substance" is a substance soluble in water, and can be said to be a water-soluble impurity such as a salt contained together with water in a solid precipitate precipitated in the medium. Here, the "salt" is a compound in which a hydrogen atom of an acid is replaced by a metal or another metallic group.

The above-described precipitation product precipitated from the medium in which the microorganism has been cultured contains water and a large amount of water-soluble impurities. Such a precipitation product further contains fat-soluble impurities and is in the form of a solid, and thus, it is difficult to sufficiently remove water-soluble substances contained in the precipitate even when the precipitation product is washed with water. However, when the extraction step using a solvent which is incompatible with water and in which the anionic biosurfactant is soluble is performed, it is possible to prepare an anionic biosurfactant composition from which the water-soluble substances have been sufficiently removed.

In the extraction step, it is not necessary to completely remove the water-soluble substances from the precipitation product serving as a target to which a solvent is added to perform extraction, and it is sufficient that the amount of the water-soluble substances in the precipitation product is reduced before and after the extraction treatment. This makes it possible to prepare the anionic biosurfactant composition having a reduced content of impurities.

As the solvent to be added to the precipitation product in the extraction step, a solvent which is incompatible with another solvent (e.g., water) capable of dissolving the water-soluble substances and in which the anionic biosurfactant is soluble can be used. Examples of such a solvent include an ester-based solvent, an alcohol-based solvent, and a hydrocarbon-based solvent, and specific examples thereof include ethyl acetate, 1-butanol, and xylene. The amount of the solvent to be added to the precipitation product is preferably from 0.1 to 10 times by mass with respect to the amount of the precipitation product, and more preferably equivalent to the amount of the precipitation product.

The extraction step will be described using a case in which ethyl acetate is used as a solvent as an example. First, a culture product is precipitated from a culture solution, ethyl acetate as a solvent is added to the precipitated precipitation product, and the mixture is sufficiently stirred to separate a solution containing the precipitation product into two layers of an ethyl acetate layer and an aqueous layer. Then, the ethyl acetate layer formed as the upper layer is separated by a separatory funnel or the like. No water-soluble substance is dissolved but an anionic biosurfactant is dissolved in the ethyl acetate layer, and thus the water-soluble substance can be removed from the precipitation product by separating the ethyl acetate layer. Then, ethyl acetate is removed from the ethyl acetate layer using, for example, an evaporator or the like, whereby a solid anionic biosurfactant composition from which the water-soluble substance has been removed can be prepared.

Note that although the case where the precipitation step and the extraction step are sequentially performed has been described above, the present invention is not necessarily limited thereto. For example, without precipitating the culture product in the medium or the culture solution, a solvent is added to the medium or the culture solution in which the product is dissolved to separate a solvent layer, whereby it is possible to prepare an anionic biosurfactant composition from which a water-soluble substance has been removed. At this time, what is prepared by removing bacterial cells and a residual substance from the culture solution in which the microorganism has been cultured by centrifugation or the like may be a target subjected to removal of the water-soluble substance using a solvent. That is, in the extraction step, the target substance from which the water-soluble substance is removed using a solvent may be a medium or a culture solution containing an anionic biosurfactant generated in the medium by a microorganism during culture, or a mixture containing a solid anionic biosurfactant separated from the reaction system.

The product containing the anionic biosurfactant prepared through the extraction step and the preceding steps will be referred to as an "extraction product".

### Fat-soluble substance removing step

The fat-soluble substance removing step is a step of removing a fat-soluble substance from the extraction product. The term "fat-soluble substance" as used herein means a substance which is soluble in an oil and/or fat.

A method for removing the fat-soluble substance from the solid extraction product from which the water-soluble substance has been removed is not particularly limited as long as it is a method capable of separating the anionic biosurfactant from the fat-soluble substance, and a method in the related art can be used. For example, first, a solvent is distilled off from the extraction product, then a solvent capable of separating an anionic biosurfactant and a fat-soluble substance is added to the solid prepared by distilling off the solvent, and the solvent layer is removed, whereby the anionic biosurfactant from which the fat-soluble substance has been removed can be prepared. This makes it possible to efficiently remove a fat-soluble impurity from the extraction product.

In the fat-soluble substance removing step, the solvent capable of separating the anionic biosurfactant and the fat-soluble substance is a solvent in which the anionic biosurfactant is slightly soluble or insoluble and the fat-soluble substance is soluble. In a case where hexane is used as such a solvent, for example, a water-soluble substance is removed from the precipitation product precipitated from the culture solution using a solvent, and then the solvent is distilled off, resulting in the formation of a solid (extraction product). The solid is suspended in hexane and filtered or centrifuged to remove hexane. This makes it possible to efficiently remove a fat-soluble impurity from the extraction product containing the anionic biosurfactant.

### Method of use

The activator according to an embodiment of the present invention may be used as an activator for various cells, but is preferably used as an activator for skin cells. Examples of skin cells to which the activator according to an embodiment of the present invention can be applied include epidermal keratinocytes and dermal fibroblasts, but the activator is preferably used for epidermal keratinocytes. In other words, the activator according to an embodiment of the present invention may be an epidermal keratinocyte activator used for epidermal keratinocytes.

### Intended use

The activator according to an embodiment of the present invention is characterized in that it has not only an activating effect but also low skin irritation and good foaming durability, and can be suitably used in cosmetics or quasi-drugs. That is, in an embodiment of the present invention, a cosmetic or quasi-drug containing the activator according to an embodiment of the present invention is provided.

Examples of the cosmetic according to an embodiment of the present invention include, but not limited to, skin cosmetics (a lotion, a serum, an emulsion, a cream, and the like), a lipstick, a sunscreen cosmetic, and a makeup cosmetic, and is preferably used in an aqueous cosmetic such as a lotion, a serum, an emulsion, a cream pack and mask, a pack, a shampoo cosmetic, a fragrance cosmetic, a liquid body cleansing agent, a UV care cosmetic, a deodorant cosmetic, or an oral care cosmetic (in a case of cosmetics).

In the present specification, the quasi-drug is a quasi-drug defined in the Pharmaceutical Affairs Act, and is defined in Article 2(2) of the Pharmaceutical Affairs Act and designated by the Minister of Health, Labour and Welfare. Specifically, among articles that are used for the purpose of diagnosis, therapy, improvement, alleviation, treatment, or prevention of diseases of humans or animals, the quasi-drug means an article having a slighter effect than that of pharmaceutical products. For example, according to the Pharmaceutical Affairs Act, a quasi-drug is an article excluding an article to be used for pharmaceutical applications, and examples thereof include a product to be used for therapy or prevention of diseases of humans or animals, a product having a slight action on the human body, and a product having no direct action on the human body. Components constituting the quasi-drug are described in the Japanese Pharmacopoeia, the Japanese Standards of Food Additives, the Japanese Industrial Standards, and the Japanese Standards of Quasi-Drug Ingredients. Specifically, the quasi-drug is used for the purpose of prevention of nausea and other unpleasant feelings, halitosis or body odor; prevention of heat rash, erosion, or the like; or prevention of hair loss, hair growth, or hair removal, and has a mild action on the human body. Specific examples of the quasi-drug include a cosmeceutical, a bath agent, an antiperspirant, a hair coloring agent, a hair growth agent, a medicated soap, and a permanent wave agent.

The content of the activator according to an embodiment of the present invention in 100 mass% of the cosmetic or quasi-drug according to an embodiment of the present invention is preferably 0.00001 mass% or more, more preferably 0.00005 mass% or more, and still more preferably 0.00001 mass or more. The upper limit of the content of the activator is preferably 10 mass% or less, more preferably 5 mass% or less, and still more preferably 3 mass% or less.

The cosmetic or quasi-drug according to an embodiment of the present invention may contain a solvent such as water and/or additional additives besides the activator according to an embodiment of the present invention.

Examples of additional additives that can be contained in the cosmetic or quasi-drug according to an embodiment of the present invention include oils and/or fats, waxes, mineral oils, fatty acids, alcohols, esters, metal soaps, gums and water-soluble macromolecular compounds, vitamins, amino acids, whitening agents, moisturizers, hair growth agents, α-hydroxy acids, inorganic pigments, ultraviolet absorbers, astringents, antioxidants, anti-inflammatory agents, bactericides and disinfectants, flavors, dyes and colorants, hormones, sequestering agents, pH adjusters, chelating agents, antiseptic and antifungal agents, cooling agents, stabilizers, animal and plant proteins and decomposition products thereof, animal and plant polysaccharides and decomposition products thereof, animal and plant glycoproteins and decomposition products thereof, blood flow promoters, anti-inflammatory and antiallergic agents, cell activators, keratolytic agents, wound healing agents, foam boosters, thickeners, oral agents, and refresher and deodorant agents.

### 2. Skin Condition Improving Agent

The anionic biosurfactant according to an embodiment of the present invention may have an enhancing effect on genes involved in skin barrier properties (e.g., CLDN1 gene, CLDN7 gene, DSC1 gene, and the like), gene which are collagen-degrading enzymes (MMP1 gene and the like), genes which are moisturizing or epidermal keratinocyte differentiation markers (e.g., LOR gene, TGM1 gene, CSTA gene, KRT1 gene, KRT4 gene, FLG gene, FLG2 gene, CASP14 gene, and the like), genes involved in hyaluronic acid generation (e.g., HAS1 gene and the like), genes involved in formation of elastin fiber which is an extracellular matrix component of the dermis (e.g., EMILIN1 gene, LOXL1 gene, and the like), genes involved in antioxidant potential (e.g., GCLC gene and the like), genes involved in antistress (e.g., HSPB1 gene and the like), and/or genes involved in improvement of skin flora (e.g., DEFB1 gene and the like). The anionic biosurfactant may also have an inhibitory effect on genes involved in proliferation of epidermal pigment cells (CXCL1 genes and the like). Accordingly, the anionic biosurfactant according to an embodiment of the present invention may have various skin condition improving effects such as a skin barrier property improving effect, a moisture retention property improving effect, an antioxidant potential promoting effect, an antistress potential improving effect, a whitening effect, a wrinkle improving effect, and a wrinkle formation inhibitory effect. Thus, the anionic biosurfactant according to an embodiment of the present invention can also be suitably used as a skin condition improving agent. That is, an embodiment of the present invention provides a skin condition improving agent including the anionic biosurfactant according to an embodiment of the present invention.

In the present specification, the term "skin condition improving agent" means a substance (composition) that improves the barrier property or moisture retention property of the skin (epidermis), inhibits changes in the skin condition due to external stimuli such as oxidative stress, inhibits formation of wrinkles by promoting degradation of collagen fibers, formation of elastin fibers, or generation of hyaluronic acid in the skin (dermis), exhibits an effect of improving already formed wrinkles, or inhibits generation of melanin, thereby exhibiting a whitening effect or inhibiting formation of macules.

More specifically, examples of the "skin condition improving agent" include a skin barrier property improving agent, a skin moisture retention improving agent, a skin wrinkle formation inhibitory agent, a skin wrinkle improving agent, a whitening promoting agent, an oxidative stress inhibitory agent, a stress inhibitory agent, and a skin bacterial flora improving agent. That is, the skin condition improving agent according to an embodiment of the present invention is preferably a skin barrier property improving agent, a skin moisture retention improving agent, a skin wrinkle formation inhibitory agent, a skin wrinkle improving agent, a whitening promoting agent, a (skin) oxidative stress inhibitory agent, a (skin) stress inhibitory agent, or a skin bacterial flora improving agent.

For a specific aspect of the anionic biosurfactant that can be contained in the skin condition improving agent according to an embodiment of the present invention, the description in the above section [1. Activator] can be referred to as appropriate. In particular, from the viewpoint of the inhibitory effect on genes (MMP1 genes and the like) that are collagen-degrading enzymes, the anionic biosurfactant contained in the skin condition improving agent according to an embodiment of the present invention is preferably an anionic biosurfactant prepared using a carbon source having 14 carbon atoms or an anionic biosurfactant prepared using a carbon source having 16 carbon atoms.

### 3. α-Gel-Containing Composition

An embodiment of the present invention provides an α-gel-containing composition including the anionic biosurfactant according to an embodiment of the present invention and a higher alcohol.

The α-gel-containing composition according to an embodiment of the present invention contains the anionic biosurfactant according to an embodiment of the present invention as a substance having surface activating ability and/or emulsifying ability, and thus exhibits effects of excellent stability and excellent sense of use when used in a cosmetic or a quasi-drug.

Note that for a specific aspect of the anionic biosurfactant according to an embodiment of the present invention contained in the α-gel-containing composition according to an embodiment of the present invention, the description in the above section [1. Activator] is referred to as appropriate.

In the present specification, the term "α-gel" refers to a gel containing an α-type hydrated crystal in which an amphiphilic substance having a hydrophilic group and a lipophilic group, such as a surfactant, forms an aggregate of bilayers stacked in a lamellar state and water is held between the hydrophilic groups. The α-gel also includes a gel in a state where water is held between hydrophilic groups of the bilayers and a large amount of water is held in a hydrophilic portion of the aggregate. The amphiphilic substance is arranged in a hexagonal shape in the bilayers, and is expected to have a high thickening effect, a high water retention effect, and a high barrier effect.

The α-gel-containing composition according to an embodiment of the present invention preferably contains 0.001 mass% or more of the anionic biosurfactant with respect to the total mass of the α-gel-containing composition. As a result, it is possible to prepare an α-gel-containing composition which is excellent in stability and is excellent in sense of use when used in a cosmetic or a pharmaceutical product (quasi-drug). The content of the anionic biosurfactant with respect to the total mass of the α-gel-containing composition is more preferably 0.005 mass% or more, still more preferably 0.010 mass% or more, still even more preferably 0.020 mass% or more, and particularly preferably 0.050 mass% or more. The upper limit of the content of the anionic biosurfactant with respect to the total mass of the α-gel-containing composition is not particularly limited, but is preferably 10.000 mass% or less, more preferably 5.000 mass% or less, and still more preferably 3.000 mass% or less.

In the α-gel-containing composition according to an embodiment of the present invention, the ratio of the content of the anionic biosurfactant to the content of the higher alcohol is preferably more than 0 and 0.500 or less, more preferably more than 0 and 0.400 or less, still more preferably more than 0.001 and 0.300 or less, and particularly preferably from 0.002 to 0.200 in terms of mass ratio. When the ratio of the content of the anionic biosurfactant to the content of the higher alcohol is within the above range, it is possible to prepare an α-gel-containing composition having excellent stability and excellent sense of use when used in a cosmetic or a pharmaceutical product (quasi-drug).

The α-gel-containing composition according to an embodiment of the present invention contains an anionic biosurfactant and a higher alcohol. In the present specification, the higher alcohol means an open-chain saturated monohydric alcohol having 6 or more carbon atoms. The higher alcohol may be a linear saturated monohydric alcohol or a branched saturated monohydric alcohol. The higher alcohol may be a primary alcohol, a secondary alcohol, or a tertiary alcohol. Examples of the higher alcohol include n-hexyl alcohol, n-heptyl alcohol, n-octyl alcohol, n-nonyl alcohol, n-decyl alcohol, n-undecyl alcohol, n-heptyl alcohol, lauryl alcohol, n-tridecyl alcohol, myristyl alcohol, n-pentadecyl alcohol, cetyl alcohol (cetanol), margaryl alcohol, stearyl alcohol, behenyl alcohol, n-nonadecyl alcohol, arachidyl alcohol, ceryl alcohol, melissyl alcohol, palmitoleyl alcohol, oleyl alcohol, eicosenyl alcohol, 2-methylpentyl alcohol, 2-ethylbutyl alcohol, 2-ethylhexyl alcohol, methylamyl alcohol, capryl alcohol, diisobutyl carbinol, isostearyl alcohol, octyldodecanol, and mixtures of two or more thereof.

Among them, the number of carbon atoms of the higher alcohol is more preferably 10 or more, still more preferably 12 or more, particularly preferably 14 or more, and most preferably 16 or more, from the viewpoint of α-gel formation. The upper limit of the number of carbon atoms is not particularly limited, and is, for example, 24 or less, more preferably 22 or less, and still more preferably 20 or less. The higher alcohol is more preferably a linear saturated monohydric alcohol from the viewpoint of α-gel formation, and is more preferably a primary alcohol from the viewpoint of α-gel formation.

In an embodiment of the present invention, more preferred examples of the higher alcohol include myristyl alcohol, cetanol, stearyl alcohol, behenyl alcohol, and a mixture of two or more thereof. These are more preferable from the viewpoint of α-gel formation.

In an embodiment of the present invention, the higher alcohol particularly preferably contains at least one of a higher alcohol having 16 carbon atoms or a higher alcohol having 18 carbon atoms. This is preferable because the sense of use is more excellent when used in a cosmetic or a pharmaceutical product (quasi-drug). Examples of such higher alcohols include cetanol or a mixture of cetanol and another higher alcohol, stearyl alcohol or a mixture of stearyl alcohol and another higher alcohol, a mixture of cetanol and stearyl alcohol, and a mixture of the mixture and another higher alcohol. In a case where the higher alcohol contains a higher alcohol having 16 carbon atoms and a higher alcohol having 18 carbon atoms, the proportion of use of the higher alcohol having 16 carbon atoms and the higher alcohol having 18 carbon atoms is preferably from 90: 10 to 10:90, more preferably from 80:20 to 20:80, still more preferably from 70:30 to 20:80, still even more preferably from 60:40 to 20:80, and particularly preferably from 55:45 to 20:80. More specific examples of such higher alcohols include a combination use of cetanol and stearyl alcohol in the above-described proportion, and cetearyl alcohol which is a mixture of cetanol and stearyl alcohol.

### Oil agent

The α-gel-containing composition according to an embodiment of the present invention may further contain an oil agent in addition to the anionic biosurfactant and the higher alcohol. When the α-gel-containing composition according to an embodiment of the present invention contains an oil agent, the oil agent can be emulsified in the gel composed of the α-type hydrated crystal and the aqueous phase to form a uniform gel composed of three phases of the α-type hydrated crystal, the aqueous phase, and the oil phase. When the uniform gel composed of three phases of the α-type hydrated crystal, the aqueous phase, and the oil phase is formed, it is possible to further improve stability of the composition, which is preferable.

As the oil agent, an oil agent which is liquid or pasty at 1 atm and 25°C is preferable, and a liquid oil agent is particularly preferable.

Examples of the liquid oil agent include waxes, hydrocarbons, higher alcohol esters, higher fatty acid esters, triglycerides, silicone oils, higher fatty acids, animal and vegetable oils, cholesterol fatty acid esters, sterols, sterol esters, and polyphenols. More preferable specific examples thereof include waxes such as carnauba wax, candelilla wax, jojoba oil, beeswax, and lanolin; hydrocarbons such as mineral oil, isododecane, squalane, vaseline, ceresin, and microcrystalline wax; esters such as isopropyl palmitate, isopropyl myristate, isooctyl myristate, isotridecyl myristate, octadecyl myristate, octyldodecyl myristate, cholesteryl isostearate, triethylhexanoin, cetyl ethylhexanoate and caprylic/capric triglyceride; dimethyl silicone oils such as octamethyltrisiloxane and dimethicone; cyclic silicone oils such as cyclotetrasiloxane, cyclopentasiloxane, cyclohexasiloxane, cyclomethicone, and methylpolycyclosiloxane; methyl phenyl silicone oils such as polyether-modified silicone oils and methylphenyl polysiloxane; mineral oils; vegetable oils such as sunflower oil, olive oil, jojoba oil, camellia oil, grapeseed oil, avocado oil, macadamia nut oil, almond oil, rice germ oil, clove oil, orange oil, and spruce oil; and triglycerides. One of these may be used alone, or two or more thereof may be used in combination.

**In** the α-gel-containing composition according to an embodiment of the present invention, which further contains the oil agent in addition to the anionic biosurfactant and the higher alcohol, the ratio of the content of the oil and/or fat to the content of the higher alcohol is preferably more than 0 and 800 or less, more preferably more than 0 and 150 or less, still more preferably more than 0 and 100 or less, and particularly preferably from 0.05 to 80.0, and may be from 0.1 to 80.0, or from 0.5 to 80.0 in terms of mass ratio. When the ratio of the content of the oil and/or fat to the content of the higher alcohol is within the above range, the stability of the α-gel-containing composition can be further improved, which is preferable.

The α-gel-containing composition according to an embodiment of the present invention more preferably further contains a polyhydric alcohol and/or a thickener to be described below in addition to the anionic biosurfactant, the higher alcohol, and the oil agent, from the viewpoint that the α-gel-containing composition is more excellent in sense of use when used in a cosmetic or a pharmaceutical product (quasi-drug).

### Polyhydric alcohol

The α-gel-containing composition according to an embodiment of the present invention may further contain a polyhydric alcohol in addition to the anionic biosurfactant and the higher alcohol. When the polyhydric alcohol is contained, the α-gel-containing composition according to an embodiment of the present invention is more excellent in the sense of use when used in a cosmetics or a pharmaceutical product (quasi-drug), which is preferable. In addition, it has been found that when the polyhydric alcohol is contained, the particle diameter of the α-gel-containing composition tends to be reduced. When the particle diameter of the α-gel-containing composition is reduced, there are further advantages that the emulsified particles are stabilized and that the compatibility with the skin is improved.

Examples of the polyhydric alcohol include, but are not limited to, propylene glycol, dipropylene glycol, 1,3-butylene glycol, pentylene glycol, glycerin, polypropylene glycol, diglycerin, polyglycerin, ethylene glycol, diethylene glycol, polyethylene glycol, 1,3-propanediol, and sorbitol. One of these may be used alone, or two or more thereof may be used in combination.

In the α-gel-containing composition according to an embodiment of the present invention, which further contains a polyhydric alcohol in addition to the anionic biosurfactant and the higher alcohol, the ratio of the content of the polyhydric alcohol to the content of the higher alcohol is preferably 0.1 or more, more preferably 1.0 or more, still more preferably 3.0 or more, and particularly preferably 5.0 or more in terms of mass ratio. The upper limit of the mass ratio is not particularly limited, but is, for example, 100.0. When the ratio of the content of the polyhydric alcohol to the content of the higher alcohol is within the above range, the stability of the α-gel-containing composition can be further improved, which is preferable.

More preferably, the α-gel-containing composition according to an embodiment of the present invention further contains the oil agent and/or a thickener described below in addition to the anionic biosurfactant, the higher alcohol, and the polyhydric alcohol, from the viewpoint that the α-gel-containing composition is more excellent in sense of use when used in a cosmetic or a pharmaceutical product (quasi-drug).

### Thickener

The α-gel-containing composition according to an embodiment of the present invention may further contain a thickener in addition to the anionic biosurfactant and the higher alcohol. When the α-gel-containing composition according to an embodiment of the present invention contains a thickener, the separation of the aqueous phase is further suppressed and the stability is improved, which is preferable.

Examples of the thickener include, but are not limited to, xanthan gum, carbomer, hydroxyethyl cellulose, methyl cellulose, ethyl cellulose, gum arabic, gellan gum, and sclerotium gum. One of these may be used alone, or two or more thereof may be used in combination.

In the α-gel-containing composition according to an embodiment of the present invention, which further contains a thickener in addition to the anionic biosurfactant and the higher alcohol, the ratio of the content of the thickener to the content of the higher alcohol is preferably 0.01 or more, more preferably 0.03 or more, still more preferably 0.05 or more, and particularly preferably 0.10 or more in terms of mass ratio. The upper limit of the mass ratio is not particularly limited, but is, for example, 10.00. When the ratio of the content of the thickener to the content of the higher alcohol is within the above range, the stability of the α-gel-containing composition can be further improved, which is preferable.

More preferably, the α-gel-containing composition according to an embodiment of the present invention further contains the oil agent and/or the polyhydric alcohol in addition to the anionic biosurfactant, the higher alcohol, and the thickener, from the viewpoint that the α-gel-containing composition is more excellent in sense of use when used in a cosmetic or a pharmaceutical product (quasi-drug).

### Additional components

The α-gel-containing composition according to an embodiment of the present invention may contain, in addition to the anionic biosurfactant, the higher alcohol, and as necessary, at least one selected from the group consisting of the oil agent, the polyhydric alcohol, and the thickener, additional components as long as the effects of the present invention are not adversely affected. Examples of the additional components include, but are not limited to, an ultraviolet absorber, an antioxidant, an emollient agent, an emulsifier, a solubilizing agent, an anti-inflammatory agent, a moisturizer, a preservative, a pH adjuster, a pigment, a flavor, and a powder. The emulsifier means an emulsifier other than the anionic biosurfactant, and for example, various anionic surfactants, cationic surfactants, amphoteric surfactants, and nonionic surfactants can be used.

The content of the additional components is not particularly limited as long as it does not adversely affect the effects of the present invention, but is, for example, from 0 to 99 mass% with respect to the total mass of the α-gel-containing composition.

### Water

The α-gel-containing composition according to an embodiment of the present invention contains the anionic biosurfactant, the higher alcohol, as necessary, at least one selected from the group consisting of the oil agent, the polyhydric alcohol, and the thickener, and as necessary, the additional components, and the remainder is water.

In an embodiment of the present invention, the amount of water contained in the α-gel-containing composition is not particularly limited, but is preferably from 1 mass% to 99 mass%, more preferably from 5 mass% to 95 mass%, and still more preferably from 10 mass% to 90 mass% with respect to the total mass of the α-gel-containing composition.

The content of water with respect to the total mass of the α-gel-containing composition is preferably 1 mass% or more from the viewpoint of α-gel formation. Furthermore, the content is preferably 99 mass% or less from the viewpoint of α-gel formation.

In an embodiment of the present invention, distilled water, ion-exchanged water, normal water, or the like can be suitably used.

### pH of α-gel-containing composition

The α-gel-containing composition according to an embodiment of the present invention preferably has a pH of 5.0 or more. When the pH is 5 or more, the stability of the α-gel-containing composition is excellent, which is preferable. The α-gel-containing composition more preferably has a pH of 6.0 or more. Furthermore, the α-gel-containing composition has a pH of preferably 12.0 or less, more preferably 11.5 or less, and still more preferably 11.0 or less.

The pH of the α-gel-containing composition according to an embodiment of the present invention can be adjusted by adding a pH adjuster during preparation of the α-gel-containing composition. Examples of the pH adjuster for adjusting the α-gel-containing composition to an alkaline side include sodium hydroxide, sodium hydrogen carbonate, potassium hydroxide, magnesium hydroxide, triethanolamine, arginine, sodium citrate, sodium lactate, and disodium succinate. Examples of the pH adjuster for adjusting the α-gel-containing composition to an acid side include citric acid, lactic acid, malic acid, and salts thereof.

### Average particle diameter of α-gel-containing composition

The average particle diameter of the α-gel-containing composition according to an embodiment of the present invention is preferably 200.0 µm or less, more preferably 100.0 µm or less, and still more preferably 50.0 µm or less.

Note that here, the average particle diameter of the α-gel-containing composition means the average particle diameter of oil particles wrapped in the lamellar aggregate of multilayers, and is a size including the multilayers.

The average particle diameter of the α-gel-containing composition is preferably 200.0 µm or less from the viewpoint of emulsion stability.

Here, the average particle diameter of the α-gel-containing composition means a median diameter (on a volume basis), and is a value measured by the method described in Examples below.

### 4. Method for Producing α-Gel-Containing Composition

A method for producing an α-gel-containing composition according to an embodiment of the present invention is not particularly limited as long as the α-gel-containing composition of interest can be prepared by the method, and various methods can be employed.

Hereinafter, the method for producing an α-gel-containing composition according to an embodiment of the present invention will be described using, as an example, an α-gel-containing composition containing an anionic biosurfactant, a higher alcohol, and an oil agent. A method for producing such an α-gel-containing composition can include an oil phase preparation step of preparing an oil phase containing an anionic biosurfactant, a higher alcohol, and an oil agent, an aqueous phase preparation step of separately preparing an aqueous phase containing water, and an emulsification step of mixing and emulsifying the aqueous phase and the oil phase.

In the oil phase preparation step, a method for preparing the oil phase containing the anionic biosurfactant, the higher alcohol, and the oil agent is not particularly limited as long as these components can be mixed. For example, a method in which the anionic biosurfactant and the higher alcohol are mixed and dissolved in each other, and then the oil agent is added to the mixture can be suitably employed. However, the order in which the components are mixed is not limited thereto, and may be any order. In addition, in the oil phase preparation step, the temperature at which the components are mixed is not particularly limited, and is any temperature as long as the components are dissolved in each other.

The aqueous phase preparation step is not particularly limited as long as it is a method in which water and, as necessary, a water-soluble component such as a polyhydric alcohol and a pH adjuster can be mixed. The order of mixing the components is not particularly limited, and may be any order. In addition, in the aqueous phase preparation step, the temperature at which the components are mixed is not particularly limited, and is any temperature as long as the components are dissolved in each other. The pH of the aqueous phase prepared in the aqueous phase preparation step is preferably 5.0 or more, and more preferably 6.0 or more.

A method of the emulsification step is not particularly limited as long as it is a step in which the aqueous phase prepared in the aqueous phase preparation step and the oil phase prepared in the oil phase preparation step can be emulsified. Preferably, a method can be exemplified in which the aqueous phase and the oil phase are mixed and stirred at 40°C to 90°C, and more preferably at 60°C to 90°C. A method for mixing the aqueous phase and the oil phase is not particularly limited, and the oil phase may be added to the aqueous phase, or the aqueous phase may be added to the oil phase. An addition method is also not particularly limited, and the aqueous phase or the oil phase may be gradually added or may be added at once. The aqueous phase and the oil phase are more preferably heated to 40°C to 90°C, and more preferably 60°C to 90°C during mixing. A method for mixing and stirring the aqueous phase and the oil phase is not particularly limited, and stirring only need be performed using a homomixer, a homodisper, a three-one motor, or the like. Among them, a homomixer is more preferably used. A stirring speed at which the aqueous phase and the oil phase are mixed and stirred is also not particularly limited, but is, for example, 1000 rpm or more, and more preferably 2000 rpm or more. A stirring time is also not particularly limited, but is, for example, 10 seconds or longer, and more preferably 1 minute or longer.

After the aqueous phase and the oil phase are emulsified in the emulsification step, the resulting α-gel-containing composition is preferably allowed to cool or cooled with a cooling device while being stirred.

### 5. Use of α-Gel-Containing Composition

The α-gel-containing composition according to an embodiment of the present invention is excellent in stability and excellent in sense of use when used in a cosmetic or a quasi-drug, and thus can be suitably used in a cosmetic or a pharmaceutical product (quasi-drug). That is, in an aspect of the present invention, a cosmetic or quasi-drug containing the α-gel-containing composition according to an embodiment of the present invention is provided.

Examples of the cosmetic or quasi-drug containing the α-gel-containing composition according to an embodiment of the present invention include, but are not limited to, skin cosmetics (a lotion, a serum, an emulsion, a cream, and the like), lipsticks, sunscreen cosmetics, and makeup cosmetics. The α-gel-containing composition according to an embodiment of the present invention is preferably used in aqueous cosmetics such as a lotion, a serum, an emulsion, a cream pack and mask, a pack, a shampoo cosmetic, a fragrance cosmetic, a liquid body cleansing agent, a UV care cosmetic, a deodorant cosmetic, or an oral care cosmetic (in the case of cosmetics).

The cosmetic or quasi-drug containing the α-gel-containing composition according to an embodiment of the present invention may contain a solvent such as water and/or additional additives besides the α-gel-containing composition according to an embodiment of the present invention.

Examples of additional additives that can be contained in the cosmetic or quasi-drug containing the α-gel-containing composition according to an embodiment of the present invention include oils and/or fats, waxes, mineral oils, fatty acids, alcohols, esters, metal soaps, gums and water-soluble macromolecular compounds, vitamins, amino acids, whitening agents, moisturizers, hair growth agents, α-hydroxy acids, inorganic pigments, ultraviolet absorbers, astringents, antioxidants, anti-inflammatory agents, bactericides and disinfectants, flavors, dyes and colorants, hormones, sequestering agents, pH adjusters, chelating agents, antiseptic and antifungal agents, cooling agents, stabilizers, animal and plant proteins and decomposition products thereof, animal and plant polysaccharides and decomposition products thereof, animal and plant glycoproteins and decomposition products thereof, blood flow promoters, anti-inflammatory and antiallergic agents, cell activators, keratolytic agents, wound healing agents, foam boosters, thickeners, oral agents, and refresher and deodorant agents.

An embodiment of the present invention may be configured as follows.
[1] An activator containing an anionic biosurfactant.
[2] The activator according to [1], wherein the anionic biosurfactant includes a biosurfactant having a glycolipid structure.
[3] The activator according to [1] or [2], wherein the anionic biosurfactant includes a succinoyl trehalose lipid.
[4] The activator according to any one of [1] to [3], wherein a content of the anionic biosurfactant is 95 parts by mass or more with respect to 100 parts by mass of the activator.
[5] The activator according to any one of [1] to [4], wherein the activator is an epidermal keratinocyte activator.
[6] A cosmetic or quasi-drug including the activator described in any one of [1] to [5].
[7] A skin condition improving agent including an anionic biosurfactant.
[8] The skin condition improving agent according to [7], wherein the skin condition improving agent is a skin barrier property improving agent.
[9] The skin condition improving agent according to [7], wherein the skin condition improving agent is a skin moisture retention improving agent.
[10] The skin condition improving agent according to [7], wherein the skin condition improving agent is a wrinkle formation inhibitory agent.
[11] The skin condition improving agent according to [7], wherein the skin condition improving agent is a wrinkle improving agent.
[12] The skin condition improving agent according to [7], wherein the skin condition improving agent is an oxidative stress inhibitory agent.
[13] The skin condition improving agent according to [7], wherein the skin condition improving agent is a stress inhibitory agent.
[14] The skin condition improving agent according to [7], wherein the skin condition improving agent is a skin bacterial flora improving agent.
[15] An α-gel-containing composition containing:
   an anionic biosurfactant; and
   a higher alcohol.
[16] The α-gel-containing composition according to [15], wherein the anionic biosurfactant includes a biosurfactant having a glycolipid structure.
[17] The α-gel-containing composition according to [15], wherein the anionic biosurfactant having a glycolipid structure is a succinoyl trehalose lipid.
[18] The α-gel-containing composition according to [15], wherein the α-gel-containing composition contains 0.001 mass% or more of the anionic biosurfactant with respect to a total mass of the α-gel-containing composition.
[19] The α-gel-containing composition according to [15], wherein the higher alcohol includes at least one of a higher alcohol having 16 carbon atoms or a higher alcohol having 18 carbon atoms.
[20] The α-gel-containing composition according to [15], wherein a ratio of a content of the anionic biosurfactant to a content of the higher alcohol is more than 0 and 0.500 or less in terms of mass ratio.
[21] The α-gel-containing composition according to [15], wherein the α-gel-containing composition has a pH of 5.0 or more.
[22] The α-gel-containing composition according to [15], further containing at least one selected from the group consisting of an oil agent, a polyhydric alcohol, and a thickener.
[23] The α-gel-containing composition according to [22], wherein the α-gel-containing composition contains an oil agent, and a ratio of a content of the oil agent to a content of the higher alcohol is more than 0 and 35.0 or less in terms of mass ratio.
[24] A cosmetic or quasi-drug containing the α-gel-containing composition described in any one of [15] to [23].

### Examples

The present invention will be described in more detail below by giving Examples, but is not limited to only these Examples. Note that "parts" represents "parts by mass" and "%" represents "mass%" unless otherwise specified.

### Example 1: Acquisition of STL C14

A Rhodococcus erythropolis strain SD-74 was subjected to seed culture in accordance with the method described in Y. Uchida et al., Agric. Biol. Chem., 53(3): 765-769 (1989) under the following conditions. The Rhodococcus erythropolis strain SD-74 used in the present Example was isolated as a vegetable oil and/or fat assimilating bacterium, and was deposited with the Patent Microorganism Depositary, National Institute of Technology and Evaluation, National Institute of Technology and Evaluation as "Accession number: NITE AP-03788".

The Rhodococcus erythropolis strain SD-74 was inoculated into 100 mL of an FPY medium (fructose (FUJIFILM Wako Pure Chemical Corporation) 2%, polypeptone (FUJIFILM Wako Pure Chemical Corporation) 0.5%, yeast extract powder (Oxoid Co., Ltd.) 0.5%, NaNO₃ (FUJIFILM Wako Pure Chemical Corporation) 0.1%, KH₂PO₄ (FUJIFILM Wako Pure Chemical Corporation) 0.1%, MgSO₄-7H₂O (FUJIFILM Wako Pure Chemical Corporation) 0.1%) in a 500 mL Sakaguchi flask and cultured with shaking at 30°C for 72 hours, resulting in the formation of a seed culture solution.

6300 mL of a modified MedD medium (per 1L, 5.44 g of KH₂PO₄ (FUJIFILM Wako Pure Chemical Corporation), 10.45 g of K₂HPO₄ (FUJIFILM Wako Pure Chemical Corporation), 3 g of KNO₃ (FUJIFILM Wako Pure Chemical Corporation), 0.1 g of MgSO₄-7H₂O (FUJIFILM Wako Pure Chemical Corporation), and 3 g of yeast extract powder (Oxoid Co., Ltd.) were diluted to 900 mL with pure water) was sterilized at high temperature and high pressure, 700 mL of tetradecane (Tokyo Chemical Industry Co., Ltd.) separately sterilized at high temperature and high pressure was added thereto, 140 mL of the seed culture solution was inoculated thereto, and main culture was started under the following conditions. Culture was performed using a 10 L jar culture tank (ABLE Corporation) at a culture temperature of 30°C with stirring at 300 rpm. Note that during the culture, the pH of the medium was maintained at 7.0 by adding 50% KOH (FUJIFILM Wako Pure Chemical Corporation).

3700 mL of the culture solution after 360 hours of the culture was centrifuged at 6500 rpm for 60 minutes to remove bacterial cells and the residual substrate in the solution, and then 150 mL of 6N HCl (FUJIFILM Wako Pure Chemical Corporation) was added thereto to adjust the pH of the solution to 2.98, whereby a white gel-like precipitate was precipitated in the solution. The solution was centrifuged at 6500 rpm for 30 minutes to remove the liquid layer, resulting in the formation of a precipitate having a wet weight of 760 g.

To the resulting precipitate, 760 g of ethyl acetate (FUJIFILM Wako Pure Chemical Corporation) was added, and the mixture was sufficiently stirred. The solution in which an aqueous layer and an ethyl acetate layer were separated was separated using a separatory funnel, and the ethyl acetate layer, which was the upper layer, was recovered. Ethyl acetate was removed from the recovered ethyl acetate solution using an evaporator. The step of suspending the solid prepared by removing ethyl acetate in an equal amount of hexane (FUJIFILM Wako Pure Chemical Corporation) and then centrifuging the suspension to remove hexane was repeated three times. The liquid prepared by removing hexane was dried by an evaporator, resulting in the formation of 100 g of a white solid (STL C14).

The STL C14 prepared in Example 1 is a succinoyl trehalose lipid having a carboxyl group generated using tetradecane as a carbon source.

### Example 2: Acquisition of STL C16

100 g of a white solid (STL C16) was prepared by the method described in Example 1 except that the carbon source described in Example 1 was changed to hexadecane (Tokyo Chemical Industry Co., Ltd.).

The STL C16 prepared in Example 2 is a succinoyl trehalose lipid having a carboxyl group generated using hexadecane as a carbon source.

### Example 3: Acquisition of STL C18

100 g of a white solid (STL C18) was prepared by the method described in Example 1 except that the carbon source described in Example 1 was changed to octadecane (Tokyo Chemical Industry Co., Ltd.).

The STL C18 prepared in Example 3 is a succinoyl trehalose lipid having a carboxyl group generated using octadecane as a carbon source.

### Cell Activation Test of STL (Test Examples 1 to 6)

The STL C14, the STL C16, and the STL C18 prepared by the methods described in Examples 1 to 3 were used as activators (i.e., an activator containing 100 parts by mass of the STL C14, the STL C16, or the STL C18 per 100 parts by mass of the activator was used) to prepare STL sample-added media in such a manner that final concentrations of the activators were the concentrations described in Table 1, and a cell activation test was performed.

A Humedia-KG2 medium (Kurabo Industries Ltd.) was used to prepare a cell suspension in such a manner that a concentration of normal human epidermal keratinocytes (Kurabo Industries Ltd.) was 8.5 × 10⁴ cells/mL, and the human epidermal keratinocytes were seeded in a 96-well multi-well plate (Thermo Fisher Scientific Inc.) at 100 µL/well. Note that the cell culture experiment was performed with 5 technical replicates. After culturing at 37°C in a 5% CO₂ atmosphere for 24 hours, the medium was discarded, and the STL sample-added medium shown in Table 1 was added at 100 µL/well.

After culturing at 37°C in a 5% CO₂ atmosphere for 48 hours, an ATP amount in each well was measured using CellTiter-Glo (trade name) 2.0 Cell Viability Assay kit (Promega) in accordance with the protocol attached to the kit, and was calculated as a relative value when an ATP amount in the non-supplemented medium was assumed to be 100. In addition, the transition of increase in the number of cells in each well was measured using Provi CM20 (EVIDENT), and a relative value was calculated when the number of cells in the non-supplemented medium was assumed to be 100. Note that Threshold = 1 and Fine Tuning = 60 were used as thresholds for the measurement of the number of cells in the Provi CM20 (EVIDENT). For the STL sample-added medium, a relative value of the ATP amount per cell was calculated in accordance with the following equation using the relative value of the ATP amount to that of the non-supplemented medium at 48 hours after the addition and the relative value of the number of cells to that of the non-supplemented medium.

Relative value of ATP amount per cell = (relative value of measured ATP amount of each well to that of non-supplemented medium)/(relative value of measured number of cells of each well to that of non-supplemented medium)

The results are shown in Table 1.

**[Table 1]**

| | Test Example 1 | Test Example 2 | Test Example 3 | Test Example 4 | Test Example 5 | Test Example 6 |
|---|---|---|---|---|---|---|
| STL used | STL C14 | | STL C16 | | STL C18 | |
| Final concentration (mass ppm) | 1 | 5 | 1 | 5 | 1 | 5 |
| Relative value of ATP amount per cell | 148 | 138.9 | 129.2 | 144.9 | 107.7 | 103.3 |

As shown in Table 1, in the samples to which the STL C14, the STL C16, or the STL C18 was added, a significant increase in ATP amount per cell was confirmed as compared with the case of the non-supplemented medium. From this, it was found that the STL C14, the STL C16, and the STL C18 had a cell activation effect.

### Cytotoxicity Test of STL

A cytotoxicity test was performed in accordance with the method described in L. Vian, J. Vinvent, J. Maurin, I. Fabre, J. Giroux, J. P. Cano, Toxicol in vitro, 9(2), pp. 185-190, 1995. Each of the STL C14, the STL C16, and the STL C18 prepared by the methods described in Examples 1 to 3 was used to prepare an STL sample-added medium in such a manner that final concentration of the STL was 1 ppm by mass, 5 ppm by mass, 10 ppm by mass, 25 ppm by mass, 50 ppm by mass, 100 ppm by mass, 250 ppm by mass, 500 ppm by mass, or 1000 ppm by mass. A DMEM medium (FUJIFILM Wako Pure Chemical Corporation) containing inactivated bovine fetus serum (final concentration 10%) was used to prepare a cell suspension in such a manner that a concentration of L929 mouse fibroblasts (KAC Co., Ltd.) was 2.0 × 10⁵ cells/mL, and the L929 mouse fibroblasts were seeded in a 96-well multi-well plate (Thermo Fisher Scientific) at 150 µL/well. Note that the cell culture experiment was performed with 3 technical replicates. After culturing at 37°C in a 5% CO₂ atmosphere for 24 hours, the STL sample-added medium was added at 150 µL/well.

After culturing at 37°C in a 5% CO₂ atmosphere for 24 hours, the medium was discarded, and a DMEM medium containing Neutral Red (FUJIFILM Wako Pure Chemical Corporation) at a final concentration of 50 µg/mL was added at 150 µL/well. After culturing at 37°C in a 5% CO₂ atmosphere for 3 hours, the medium was discarded, and cells were washed with a D-PBS(-) solution (FUJIFILM Wako Pure Chemical Corporation). Ethanol (FUJIFILM Wako Pure Chemical Corporation) was used to prepare 50% ethanol aqueous solution, and glacial acetic acid (FUJIFILM Wako Pure Chemical Corporation) was added to the 50% ethanol aqueous solution to achieve a final concentration of 1%. This 1% glacial acetic acid ethanol aqueous solution was added at 100 µL/well to fix the cells. After 5 minutes, an absorbance at 546 nm was measured using a corona multi-grating microplate reader SH-9000Lab (Hitachi High-Tech Science Corporation), and living cells having incorporated Neutral Red were quantified. A 50% cell growth inhibitory concentration (IC50) was calculated as the evaluation score of the cytotoxicity. Note that for the STL C18, 50% cell growth inhibition was not observed even in 1000 ppm which was the highest STL concentration tested in this experiment.

For the IC50 of sodium lauryl sulfate, sodium deoxycholate, and sodium caprylate, the values of L. Vian, J. Vinvent, J. Maurin, I. Fabre, J. Giroux, J. P. Cano, Toxicol in vitro, 9(2), pp. 185-190, 1995 are employed.

The results are described in Table 4.

The STL C14, the STL C16, and the STL C18 all had a value of IC50 equal to or more than 300 ppm and were found to be less cytotoxic.

### Physiological Function Evaluation Test of STL

The STL C14, the STL C16, and the STL C18 prepared by the methods described in Examples 1 to 3 were used as activators for various physiological functions (that is, an activator containing 100 parts by mass of the STL C14, the STL C16, or the STL C18 per 100 parts by mass of the activator was used) to prepare STL sample-added media in such a manner that the final concentrations of the activators were a concentration of 10 ppm, and a physiological function evaluation test was performed.

A Humedia-KG2 medium (Kurabo Industries Ltd.) was used to prepare a cell suspension in such a manner that a concentration of normal human epidermal keratinocytes (Kurabo Industries Ltd.) was 1.53 × 10⁵ cells/mL, and the human epidermal keratinocytes were seeded in 4-Well Rectangular Dish, TC Surface (Thermo Fisher Scientific) at 5 mL/well. Note that the cell culture experiment was performed with 3 technical replicates. After culturing at 37°C in a 5% CO₂ atmosphere for 24 hours, the medium was discarded, and a medium prepared by adding any of the activators (an activator containing 100 parts by mass of the STL C14, the STL C16, or the STL C18 per 100 parts by mass of the activator) to a Humedia-KG2 medium (Kurabo Industries Ltd.) in such a manner that the final concentration was 10 ppm (STL C14-added group, STL C16-added group, and STL C18-added group), or a Humedia-KG2 medium (Kurabo Industries Ltd.) as a non-added group was added to a 4-Well Rectangular Dish, TC Surface (Thermo Fisher Scientific) at 5 mL/well.

After culturing at 37°C in a 5% CO₂ atmosphere for 24 hours, the medium was discarded, RNAprotect Cell Reagen (Qiagen) was added at 3 mL/well, and cells were recovered. Total RNA was extracted from the recovered cells using miRNeasy Mini Kit (Qiagen) in accordance with the protocol attached to the kit. NanoDrop One (Thermo Fisher Scientific) and TapeStation (Agilent Technologies) were used to measure a purity and a degree of degradation of RNA, and it was confirmed that both of the 260/280 ratio and the 260/230 ratio, which are indexes of the purity of RNA, were 1.9 or more, and an RIN value, which is an index of the degree of degradation of RNA, was 8 or more. After an RNA concentration was measured using Quant-iT (trade name) RNAAssay Kits (Thermo Fisher Scientific), total RNA was added using ReverTra Ace qPCR RT master mix (TOYOBO Co., Ltd.) in such a manner that the final RNA concentration was 50 ng/µL, thereby performing a reverse transcription reaction, resulting in the formation of cDNA.

A QIAcuity probe PCR kit (Qiagen), a QIAcuity Nanoplate 8.5k 96-well (Qiagen), a QIAcuity Four digital PCR system (Qiagen), and a Tagman probe (Thermo Fisher Scientific) described in Table 2 below were used to perform a quantitative PCR experiment in accordance with the protocol published by Qiagen, and an average value of relative values of each gene was calculated, taking the value of the non-added group as 1. Note that the quantitative PCR experiment was performed with 2 technical replicates. The results of gene expression analysis by the quantitative PCR are shown in Table 3.

**[Table 2]**

| (Table 2) Tagman probe used for gene expression analysis | |
|---|---|
| Gene name | Product name of Thermo Fischer Scientific |
| CLDN1 | Hs00221623_m1 |
| CLDN7 | Hs00600772_m1 |
| DSC1 | Hs00245189_m1 |
| LOR | Hs01894962_s1 |
| TGM1 | Hs00165929_m1 |
| CSTA | Hs00193257_m1 |
| KRT1 | Hs00196158_m1 |
| KRT4 | Hs00361611_m1 |
| FLG | Hs00856927_g1 |
| FLG2 | Hs00418578_m1 |
| CASP14 | Hs00201637_m1 |
| CXCL1 | Hs00236937_m1 |
| HAS1 | Hs00758053_m1 |
| EMILIN1 | Hs00918337_g1 |
| LOXL1 | Hs00935937_m1 |
| MMP1 | Hs00899658_m1 |
| GCLC | Hs00155249_m1 |
| HSPB1 | Hs00356629_g1 |
| DEFB1 | Hs00608345_m1 |

**[Table 3]**

| (Table 3) Results of gene expression analysis by quantitative PCR | | | |
|---|---|---|---|
| Gene name | Added sample name | Relative value (average) when value of non-added group is taken as 1 | Expected effect |
| CLDN1 | STL C14 | 3.17 | |
| | STL C16 | 1.25 | |
| | STL C18 | 1.48 | |
| CLDN7 | STL C14 | 2.28 | Skin barrier property improvement |
| | STL C16 | 1.34 | |
| | STL C18 | 1.72 | |
| DSC1 | STL C14 | 3.05 | |
| | STL C16 | 1.58 | |
| | STL C18 | 1.67 | |
| LOR | STL C14 | 3.00 | |
| | STL C16 | 2.33 | |
| | STL C18 | 1.70 | |
| TGM1 | STL C14 | 1.79 | |
| | STL C16 | 1.31 | |
| | STL C18 | 1.78 | |
| CSTA | STL C14 | 2.37 | |
| | STL C16 | 1.29 | |
| | STL C18 | 1.27 | Keratinocyte differentiation promoting effect and moisture retention improvement |
| KRT1 | STL C14 | 4.34 | |
| | STL C16 | 2.08 | |
| | STL C18 | 1.21 | |
| KRT4 | STL C14 | 2.19 | |
| | STL C16 | 1.39 | |
| | STL C18 | 1.01 | |
| FLG | STL C14 | 2.98 | |
| | STL C16 | 2.40 | |
| | STL C18 | 1.16 | |
| FLG2 | STL C14 | 4.20 | |
| | STL C16 | 1.97 | |
| | STL C18 | 0.97 | |
| CASP14 | STL C14 | 6.85 | |
| | STL C16 | 1.76 | |
| | STL C18 | 1.69 | |

**(Continuation of Table 3)**

| | | | |
|---|---|---|---|
| CXCL1 | STL C14 | 0.06 | Whitening effect |
| | STL C16 | 0.54 | |
| | STL C18 | 0.87 | |
| HAS1 | STL C14 | 6.82 | |
| | STL C16 | 3.54 | |
| | STL C18 | 1.46 | |
| EMILIN 1 | STL C14 | 5.13 | |
| | STL C16 | 2.44 | |
| | STL C18 | 1.95 | Wrinkle improving effect, wrinkle formation inhibitory effect |
| LOXL1 | STL C14 | 3.35 | |
| | STL C16 | 1.86 | |
| | STL C18 | 2.10 | |
| MMP1 | STL C14 | 0.05 | |
| | STL C16 | 0.79 | |
| | STL C18 | 2.24 | |
| GCLC | STL C14 | 4.05 | Antioxidative effect |
| | STL C16 | 1.28 | |
| | STL C18 | 1.17 | |
| HSPB1 | STL C14 | 3.47 | Anti-stress effect |
| | STL C16 | 1.27 | |
| | STL C18 | 1.19 | |
| DEFB1 | STL C14 | 4.37 | Skin flora improving effect |
| | STL C16 | 1.76 | |
| | STL C18 | 2.13 | |

As a result of the gene expression analysis by the quantitative PCR, the following were confirmed in the STL C14-added group, the STL C16-added group, and the STL C18-added group: the expression levels of CLDN1, CLDN7, and DSC1 genes related to skin barrier properties were increased; LOR, TGM1, CSTA, KRT1, KRT4, FLG, FLG2, and CASP14 genes related to moisture retention and serving as epidermal keratinocyte differentiation markers were increased; a CXCL1 gene related to epidermal pigment cell proliferation was inhibited; an HAS 1 gene related to hyaluronic acid generation was increased; EMILIN1 and LOXL 1 genes related to elastin fiber formation, which is a dermal extracellular matrix component, were promoted; a GCLC gene related to antioxidant potential was increased; and an HSPB 1 gene related to antistress and a DEFB 1 gene related to improvement of skin flora were increased. In addition, in the STL C14-added group and the STL C16-added group, an inhibitory effect on an MMP1 gene, which is a collagen-degrading enzyme, was confirmed. From the above, it was found that the STL C14, the STL C16, and the STL C18 can be expected to have a skin barrier property improving effect, a moisture retention improving effect, an antioxidant potential promoting effect, an antistress potential improving effect, a whitening effect, a wrinkle improving effect, and a wrinkle formation inhibitory effect.

### Measurement of Critical Micelle Concentration (CMC) of STL

A highly functional surface tensiometer (DY-500 (available from Kyowa Interface Science Co., Ltd.) was used to perform surface tension measurement by a platinum suspension plate method, and critical micelle concentrations (CMCs) of the STL C14, the STL C16, the STL C18, sodium lauryl sulfate, sodium deoxycholate, and sodium caprylate were calculated in accordance with a common method. The results are described in Table 4.

The CMCs of the STL C14, the STL C16, and the STL C18 were all low values, and thus it was found that the STL C14, the STL C16, and the STL C18 had high surface activating ability.

### Protein Denaturation Test of STL

The STL C14, the STL C16, and the STL C18 prepared in Examples 1 to 3 were used to prepare 10 mL of aqueous solutions (pH 8.0) in such a manner that the final STL concentration was 0.5%. Note that the pH was adjusted using an aqueous sodium hydroxide solution (FUJIFILM Wako Pure Chemical Corporation). 0.03 g of Zein powder (FUJIFILM Wako Pure Chemical Corporation) was added to the aqueous solutions of the STL. After stirring at room temperature and 1200 rpm for 2 hours, the Zein powder-added solutions were transferred to centrifuge tubes, respectively, and centrifuged at 6200 rpm for 10 seconds to precipitate and remove undissolved zein powder. The protein concentrations in the solutions were measured using a microvolume spectrophotometer NanoDrop (NanoDrop Technologies). The results are described in Table 4.

The protein concentrations in the 0.5% aqueous solutions of the STL C14, the STL C16, and the STL C18 were low, and thus it can be determined that the added proteins were not denatured. From this, it was found that the protein-denaturing abilities of the STL C14, the STL C116, and the STL C18 were low. The amount of denatured protein is generally considered to be proportional to skin irritation, and thus it was suggested that the STL C14, the STL C16, and the STL C18 had low skin irritation.

The protein denaturation test was performed in the same manner using sodium lauryl sulfate, sodium deoxycholate, and sodium caprylate. The results are described in Table 4.

### Foam Property Evaluation of STL

The STL C14, the STL C16, and the STL C18 prepared in Examples 1 to 3 and water having a hardness of 20°dH were used to prepare 10 mL of aqueous solutions (pH 8.0) in such a manner that the final concentration of each of STLs was 0.5%. Note that for pH adjustment, an aqueous sodium hydroxide solution (FUJIFILM Wako Pure Chemical Corporation) was used. Each of the prepared aqueous solutions was stirred at 25°C for 100 seconds using a dynamic foam analyzer (DFA100, available from KRUSS Scientific Instruments), and images of foams were taken 20 seconds and 600 seconds after the stirring was stopped. Thereafter, an average bubble area of foams was determined based on the images, and creaminess (foamability) of the foams was evaluated according to the following criteria. The results are described in Table 4. Note that the creaminess of the foams is characterized by having a small final bubble diameter (measured as average bubble area). The foams generated from the aqueous solutions containing the STL C14, the STL C16, or the STL C18 had small bubble areas, and thus it was considered that creamy foams could be formed.

### Evaluation criteria for creaminess of foams

⊚ (good): The bubble area was 20000 µm² or less.
∘ (passed): The bubble area was 20001 µm² or more and 30000 µm² or less.
Δ (slightly poor): The bubble area was 30001 µm² or more and 40000 µm² or less.
× (poor): The bubble area was 40001 µm² or more.

A foam change rate was calculated using the following equation. It can be determined that the smaller the foam change rate is, the better the foam retention is, in other words, the better the foaming durability is. In the STL C14, the STL C16, and the STL C18, the foam change rate was small, and thus it was considered that it was possible to form foams having good foam retention. Foam change rate = (average bubble area after 20 seconds from stirring stop)/(average bubble area after 600 seconds from stirring stop)

The foam property evaluation was similarly performed using sodium lauryl sulfate, sodium deoxycholate, and sodium caprylate. The results are described in Table 4.

**[Table 4]**

| | Example 1 | Example 2 | Example 3 | | | |
|---|---|---|---|---|---|---|
| | STL C14 | STL C16 | STL C18 | Sodium lauryl sulfate | Sodium deoxycholate | Sodium caprylate |
| Cytotoxicity evaluation IC50 (ppm) | 344 | 541 | 1000 or more | 86 | 90 | 887 |
| Surface activating ability evaluation CMC (× 10⁻⁵ M) | 2.2 | 6.5 | 12 | 800 | 600 | 30000 |
| Protein denaturation ability evaluation Protein dissolution concentration (µg/mL) | 60 | 70 | 40 | 1490 | 430 | 170 |
| Creaminess of foam Bubble area after 20 seconds | ○ | ⊚ | ⊚ | ⊚ | ⊚ | ○ |
| Creaminess of foam Bubble area after 600 seconds | ⊚ | ⊚ | ⊚ | Δ | × | Δ |
| Foam retention evaluation Foam change rate (%) | 0.53 | 1.03 | 0.75 | 2.81 | 2.62 | 1.09 |

From Examples 1 to 3, it was found that the anionic biosurfactant was useful as a cell activator. It was also found that the above-described anionic biosurfactant had low skin irritation and good foaming durability. It was also found that the above-described anionic biosurfactant was useful as a skin condition improving agent.

### Example 4

0.100 parts by mass of the anionic biosurfactant prepared in Example 1 and 3.000 parts by mass of cetearyl alcohol as a higher alcohol were mixed at 80°C to 85°C, resulting in the formation of a solution in which the anionic biosurfactant prepared in Example 1 was dissolved in the higher alcohol. To the resulting solution, 50.000 parts by mass of squalane was added as an oil agent, and the mixture was heated to 80°C to 85°C to prepare an oil phase.

Separately, 46.590 parts by mass of purified water was mixed with 0.013 parts by mass of sodium hydroxide, and the mixture was heated to 80°C to 85°C to prepare an aqueous phase.

While stirring using a homomixer at 3000 rpm, the oil phase was gradually added to the aqueous phase, and after the total amount was added, the mixture was stirred at 5000 rpm for 5 minutes. Immediately after the mixture was emulsified, 10.000 parts by mass of xanthan gum as a 2% aqueous solution (0.200 parts by mass as xanthan gum) was added thereto as a thickener. Thereafter, the mixture to which the thickener was added was cooled to 35°C by natural cooling while being stirred at 1000 rpm, resulting in the formation of a gel-like composition. The X-ray diffraction pattern confirmed that the resulting gel-like composition was an α-gel-containing composition.

### Example 5

A gel-like composition was prepared by performing the same operation as in Example 4 except that 2.000 parts by mass of cetearyl alcohol and 1.000 parts by mass of behenyl alcohol were used as the higher alcohol instead of 3.000 parts by mass of cetearyl alcohol.

### Example 6

A gel-like composition was prepared by performing the same operation as in Example 4 except that 2.100 parts by mass of cetanol and 0.900 parts by mass of stearyl alcohol were used as the higher alcohol instead of 3.000 parts by mass of cetearyl alcohol. The X-ray diffraction pattern confirmed that the resulting gel-like composition was an α-gel-containing composition.

### Example 7

A gel-like composition was prepared by performing the same operation as in Example 4 except that 50.000 parts by mass of cetyl 2-ethylhexanoate was used as the oil agent instead of 50.000 parts by mass of squalane.

### Example 8

A gel-like composition was prepared by performing the same operation as in Example 4 except that 50.000 parts by mass of dimethicone was used as the oil agent instead of 50.000 parts by mass of squalane. The X-ray diffraction pattern confirmed that the resulting gel-like composition was an α-gel-containing composition.

### Example 9

A gel-like composition was prepared by performing the same operation as in Example 4 except that 0.500 parts by mass of microcrystalline wax was used as the oil agent in addition to 50.000 parts by mass of squalane, and the blending amount of purified water was accordingly changed as shown in Table 5.

### Example 10

A gel-like composition was prepared by performing the same operation as in Example 4 except that the amount of squalane used as the oil agent was changed from 50.000 parts by mass to 30.000 parts by mass, and the blending amount of purified water was accordingly changed as shown in Table 5. The X-ray diffraction pattern confirmed that the resulting gel-like composition was an α-gel-containing composition.

### Example 11

A gel-like composition was prepared by performing the same operation as in Example 4 except that 10.000 parts by mass of 1,3-butylene glycol (polyhydric alcohol) was added in the preparation of the aqueous phase, and the blending amount of purified water was accordingly changed as shown in Table 5.

Specifically, 36.590 parts by mass of purified water was mixed with 0.013 parts by mass of sodium hydroxide and 10.000 parts by mass of 1,3-butylene glycol (polyhydric alcohol), and the mixture was heated to 80°C to 85°C to prepare an aqueous phase.

The X-ray diffraction pattern confirmed that the resulting gel-like composition was an α-gel-containing composition.

### Example 12

A gel-like composition was prepared by performing the same operation as in Example 11 except that 10.000 parts by mass of 1,3-butylene glycol (polyhydric alcohol) was changed to 10.000 parts by mass of glycerin (polyhydric alcohol).

### Example 13

A gel-like composition was prepared by performing the same operation as in Example 11 except that 10.000 parts by mass of 1,3-butylene glycol (polyhydric alcohol) was changed to 10.000 parts by mass of pentylene glycol (polyhydric alcohol).

### Example 14

A gel-like composition was prepared by performing the same operation as in Example 8 except that 10.000 parts by mass of 1,3-butylene glycol (polyhydric alcohol) was added in the preparation of the aqueous phase, and the blending amount of purified water was accordingly changed as shown in Table 5.

Specifically, 36.590 parts by mass of purified water was mixed with 0.013 parts by mass of sodium hydroxide and 10.000 parts by mass of 1,3-butylene glycol (polyhydric alcohol), and the mixture was heated to 80°C to 85°C to prepare an aqueous phase.

The X-ray diffraction pattern confirmed that the resulting gel-like composition was an α-gel-containing composition.

### Example 15

A gel-like composition was prepared by performing the same operation as in Example 4 except that 0.100 parts by mass of glyceryl stearate (nonionic surfactant) was used as the surfactant in addition to 0.100 parts by mass of the anionic biosurfactant prepared in Example 1, and the blending amount of purified water was accordingly changed as shown in Table 6.

Specifically, in the preparation of the oil phase, 0.100 parts by mass of the anionic biosurfactant prepared in Example 1, 0.100 parts by mass of glyceryl stearate, and 3.000 parts by mass of cetearyl alcohol were mixed, resulting in the formation of a solution in which the surfactant was dissolved in a higher alcohol. 50.000 parts by mass of squalane as an oil agent was added to the resulting solution, followed by heating to 80°C to 85°C.

### Example 16

A gel-like composition was prepared by performing the same operation as in Example 4 except that the blending amount of the anionic biosurfactant prepared in Example 1 was changed from 0.100 parts by mass to 0.300 parts by mass and the blending amounts of purified water and sodium hydroxide were accordingly changed as shown in Table 6. The X-ray diffraction pattern confirmed that the resulting gel-like composition was an α-gel-containing composition.

### Example 17

A gel-like composition was prepared by performing the same operation as in Example 4 except that the blending amount of the anionic biosurfactant prepared in Example 1 was changed from 0.100 parts by mass to 0.030 parts by mass and the blending amounts of purified water and sodium hydroxide were accordingly changed as shown in Table 6. The X-ray diffraction pattern confirmed that the resulting gel-like composition was an α-gel-containing composition.

### Example 18

A gel-like composition was prepared by performing the same operation as in Example 4 except that the blending amounts of the anionic biosurfactant prepared in Example 1 and the higher alcohol were changed to 1.5 times and the blending amounts of the purified water and sodium hydroxide were accordingly changed as shown in Table 6.

Specifically, the blending amount of the anionic biosurfactant prepared in Example 1 was changed from 0.100 parts by mass to 0.150 parts by mass, and the blending amount of cetearyl alcohol was changed from 3.000 parts by mass to 4.500 parts by mass. Accordingly, the blending amount of purified water was changed from 46.590 parts by mass to 45.030 parts by mass, and the blending amount of sodium hydroxide was changed from 0.013 parts by mass to 0.020 parts by mass.

### Example 19

A gel-like composition was prepared by performing the same operation as in Example 4 except that the thickener (0.200 parts by mass of xanthan gum) was not used and the blending amount of purified water was accordingly changed as shown in Table 6.

### Example 20

A gel-like composition was prepared by performing the same operation as in Example 4 except that carbomer neutralized with sodium hydroxide (prepared by adding 0.100 parts by mass of sodium hydroxide to 10.000 parts by mass of a 2% aqueous solution of carbomer (0.200 parts by mass as carbomer)) was added as a thickener instead of 0.200 parts by mass of xanthan gum. The total blending amount of sodium hydroxide is accordingly as shown in Table 6.

### Example 21

A gel-like composition was prepared by performing the same operation as in Example 4 except that 0.029 parts by mass of citric acid was further added in the preparation of the aqueous phase and the blending amount of purified water was accordingly changed as shown in Table 6.

Specifically, 46.560 parts by mass of purified water was mixed with 0.013 parts by mass of sodium hydroxide and 0.029 parts by mass of citric acid and the mixture was heated to 80°C to 85°C to prepare an aqueous phase.

### Example 22

A gel-like composition was prepared by performing the same operation as in Example 21 except that the blending amount of citric acid was changed from 0.029 parts by mass to 0.016 parts by mass.

### Example 23

A gel-like composition was prepared by performing the same operation as in Example 21 except that the blending amount of citric acid was changed from 0.029 parts by mass to 0.009 parts by mass.

### Comparative Example 1

A gel-like composition was prepared by performing the same operation as in Example 4 except that 0.100 parts by mass of the anionic biosurfactant prepared in Example 1 was not added and the blending amount of purified water was accordingly changed as shown in Table 6. The X-ray diffraction pattern confirmed that the resulting gel-like composition contained no α-gel.

### Comparative Example 2

A gel-like composition was prepared by performing the same operation as in Example 4 except that 0.100 parts by mass of the anionic biosurfactant prepared in Example 1 was changed to 0.100 parts by mass of glyceryl stearate (nonionic surfactant).

### Evaluation of Gel-like Composition

Evaluation of whether or not the gel-like compositions prepared in Examples and Comparative Examples were α-gel-containing compositions, measurement of the average particle diameter, and evaluation of stability and sense of use were performed by the methods described below.

### Evaluation of whether or not composition is α-gel-containing composition

XRD measurement was performed under the following conditions, and α-gel formation was determined from presence or absence of a sharp peak between 21° and 22° in a wide angle region. Specifically, in a case where a sharp peak was present between 21° and 22° in the wide angle region, the composition was determined to be an α-gel-containing composition.

### XRD Measurement

Device: Smart Lab (available from Rigaku Corporation)
X-ray source: Cu K-α (1.5405 Å)
Tube voltage: 45 kV
Tube current: 200 mA

### Average particle diameter

The average particle diameter was measured as a median diameter (volume-based) using a particle size measuring apparatus LA-950V2 available from HORIBA. The measurement was performed at room temperature.

### Stability evaluation (1)

Each of the gel-like compositions prepared in Examples and Comparative Examples was placed in a glass container with a lid, sealed, and allowed to stand at 25°C for 1 hour. Then, the state of the gel-like composition was observed and evaluated according to the following criteria. Note that the term "glossy" refers to a state in which the gel-like composition is glossy.
A: No oil phase separation on the upper surface, glossy
B: No oil phase separation on the upper surface, not glossy
C: Slight oil phase separation on the upper surface
D: The oil phase is separated on the upper surface.

### Stability evaluation (2)

Each of the gel-like compositions prepared in Examples and Comparative Examples was placed in a glass container with a lid, sealed, and allowed to stand at 50°C for 1 week. Then, the state of the gel-like composition was observed and evaluated according to the following criteria.
A: No lower phase separation (separation of the aqueous phase occurring at the lower portion)
D: Lower phase separation (separation of the aqueous phase occurring in the lower portion).

### Sense of use

Sense of use was evaluated by five expert panelists. Specifically, each of the gel-like compositions prepared in Examples and Comparative Examples was applied onto the skin of the inner part of the forearm, and the sense of use was evaluated.

Evaluation was performed according to the following criteria. The wording "having rich feeling" refers to a state in which thickness and resistance feeling are felt at the time of application. The evaluation results of the five panelists were averaged and rounded off to give a value of "sense of use".
5: Having very rich feeling
4: Having rich feeling
3: Having slightly rich feeling
2: Having almost no rich feeling
1: Having no rich feeling.

### Evaluation results

The evaluation results are shown in the following Tables 5 and 6 together with the blending amounts of the respective components. In the tables, the unit of the blending amount is parts by mass. In Table 5, for a portion where no median diameter is described, the median diameter was not measured. In Comparative Example 1, the gel-like composition was separated, and thus the sense of use was not evaluated.

### [Table 5]

**(Table 5)**

| | | Example 4 | Example 5 | Example 6 | Example 7 | Example 8 | Example 9 | Example 10 | Example 11 | Example 12 | Example 13 | Example 14 |
|---|---|---|---|---|---|---|---|---|---|---|---|---|
| Surfactant | STL C14 | 0.100 | 0.100 | 0.100 | 0.100 | 0.100 | 0.100 | 0.100 | 0.100 | 0.100 | 0.100 | 0.100 |
| | Glyceryl stearate | - | - | - | - | - | - | - | - | - | - | - |
| pH adjuster | Sodium hydroxide | 0.013 | 0.013 | 0.013 | 0.013 | 0.013 | 0.013 | 0.013 | 0.013 | 0.013 | 0.013 | 0.013 |
| | Citric acid | - | - | - | - | - | - | - | - | - | - | - |
| Higher alcohol | Cetearyl alcohol | 3.000 | 2.000 | - | 3.000 | 3.000 | 3.000 | 3.000 | 3.000 | 3.000 | 3.000 | 3.000 |
| | Cetanol | - | - | 2.100 | - | - | - | - | - | - | - | - |
| | Stearyl alcohol | - | - | 0.900 | - | - | - | - | - | - | - | - |
| | Behenyl alcohol | - | 1.000 | - | - | - | - | - | - | - | - | - |
| Oil agent | Microcrystalline wax | - | - | - | - | - | 0.500 | - | - | - | - | - |
| | Dimethicone | - | - | - | - | 50.000 | - | - | - | - | - | 50.000 |
| | Squalane | 50.000 | 50.000 | 50.000 | - | - | 50.000 | 30.000 | 50.000 | 50.000 | 50.000 | - |
| | Cetyl ethylhexanoate | - | - | - | 50.000 | - | - | - | - | - | - | - |
| Polyhydric alcohol | 1,3-butylene glycol | - | - | - | - | - | - | - | 10.000 | - | - | 10.000 |
| | Glycerin | - | - | - | - | - | - | - | - | 10.000 | - | - |
| | Pentylene glycol | - | - | - | - | - | - | - | - | - | 10.000 | - |
| Thickener | Xanthan gum | 0.200 | 0.200 | 0.200 | 0.200 | 0.200 | 0.200 | 0.200 | 0.200 | 0.200 | 0.200 | 0.200 |
| | Carbomer | - | - | - | - | - | - | - | - | - | - | - |
| Water | Purified water | 46.687 | 46.687 | 46.687 | 46.687 | 46.687 | 46.187 | 66.687 | 36.687 | 36.687 | 36.687 | 36.687 |
| Evaluation result | pH | 8.0 | 8.0 | 8.0 | 8.0 | 8.0 | 8.0 | 8.0 | 8.0 | 8.0 | 8.0 | 8.0 |
| | Stability evaluation (1) | A | A | A | A | A | A | A | A | A | A | A |
| | Sense of use | 4 | 3 | 3 | 3 | 4 | 4 | 3 | 5 | 5 | 5 | 5 |
| | Median diameter (µm) | 22.5 | 17.7 | - | - | 20.6 | - | - | 14.4 | 12.8 | - | 8.3 |
| | Stability evaluation (2) | A | - | - | - | - | - | - | - | - | - | - |

### [Table 6]

**(Table 6)**

| | | Example 15 | Example 16 | Example 17 | Example 18 | Example 19 | Example 20 | Example 21 | Example 22 | Example 23 | Comparative Example 1 | Comparative Example 2 |
|---|---|---|---|---|---|---|---|---|---|---|---|---|
| Surfactant | STL C14 | 0.100 | 0.300 | 0.030 | 0.150 | 0.100 | 0.100 | 0.100 | 0.100 | 0.100 | - | - |
| | Glyceryl stearate | 0.100 | - | - | - | - | - | - | - | - | - | 0.100 |
| pH adjuster | Sodium hydroxide | 0.013 | 0.039 | 0.004 | 0.020 | 0.013 | 0.113 | 0.013 | 0.013 | 0.013 | 0.013 | 0.013 |
| | Citric acid | - | - | - | - | - | - | 0.029 | 0.016 | 0.009 | - | - |
| Higher alcohol | Cetearyl alcohol | 3.000 | 3.000 | 3.000 | 4.500 | 3.000 | 3.000 | 3.000 | 3.000 | 3.000 | 3.000 | 3.000 |
| | Cetanol | - | - | - | - | - | - | - | - | - | - | - |
| | Stearyl alcohol | - | - | - | - | - | - | - | - | - | - | - |
| | Behenyl alcohol | - | - | - | - | - | - | - | - | - | - | - |
| Oil agent | Microcrystalline wax | - | - | - | - | - | - | - | - | - | - | - |
| | Dimethicone | - | - | - | - | - | - | - | - | - | - | - |
| | Squalane | 50.000 | 50.000 | 50.000 | 50.000 | 50.000 | 50.000 | 50.000 | 50.000 | 50.000 | 50.000 | 50.000 |
| | Cetyl ethylhexanoate | - | - | - | - | - | - | - | - | - | - | - |
| Polyhydric alcohol | 1,3-butylene glycol | - | - | - | - | - | - | - | - | - | - | - |
| | Glycerin | - | - | - | - | - | - | - | - | - | - | - |
| | Pentylene glycol | - | - | - | - | - | - | - | - | - | - | - |
| Thickener | Xanthan gum | 0.200 | 0.200 | 0.200 | 0.200 | - | - | 0.200 | 0.200 | 0.200 | 0.200 | 0.200 |
| | Carbomer | - | - | - | - | - | 0.200 | - | - | - | - | - |
| Water | Purified water | 46.587 | 46.461 | 46.766 | 45.13 | 46.887 | 46.787 | 46.658 | 46.671 | 46.678 | 46.787 | 46.687 |
| Evaluation result | pH | 8.0 | 8.0 | 8.0 | 8.0 | 8.0 | 8.0 | 5.3 | 6.3 | 7.1 | 8.0 | 8.0 |
| | Stability evaluation (1) | A | A | A | A | A | A | B | A | A | D | C |
| | Sense of use | 4 | 3 | 3 | 3 | 4 | 4 | 3 | 3 | 4 | - | 2 |
| | Stability evaluation (2) | - | - | - | - | D | A | - | - | - | - | - |

### Summary

From Examples 4 to 10, it was confirmed that a gel-like composition containing an anionic biosurfactant and a higher alcohol forms an α-gel. In addition, the resulting α-gel-containing composition was superior in stability and sense of use to the gel-like composition prepared by using glyceryl stearate (nonionic surfactant) instead of the anionic biosurfactant under the same conditions.

Furthermore, the α-gel-containing compositions prepared in Examples 11 to 14, in which a polyhydric alcohol was used in addition to an anionic biosurfactant and a higher alcohol, were very excellent in sense of use. In addition, the α-gel-containing compositions prepared in Examples 11 to 14, in which the polyhydric alcohol was used in addition to the anionic biosurfactant and the higher alcohol, had a small average particle diameter, indicating that the particle diameter of the α-gel-containing composition was reduced. Furthermore, from the comparison between Example 19 and Example 20, it was shown that in the α-gel-containing composition containing a thickener, the separation of the aqueous phase was further suppressed to improve the stability.

### Formulation Examples

Emulsified compositions of Formulation Examples 1 to 4 shown in Table 7 below were prepared using the anionic biosurfactant prepared in Example 1.

### Adjustment method

An oil phase 1 and an oil phase 2 were prepared by mixing and dissolving at 80°C to 85°C in advance, and the mixture of the two was used as the oil phase. Separately, an aqueous phase was prepared by heating and mixing at 80°C to 85°C. Sodium hydroxide was added to achieve the pH of the aqueous phase of 8.

While stirring using a homomixer at 3000 rpm, the oil phase was gradually added to the aqueous phase, and after the total amount was added, the mixture was stirred at 5000 rpm for 5 minutes. Thereafter, while stirring at 1000 rpm, when the mixture was cooled to 55°C by natural cooling, Additive 1 and Additive 2 were added thereto and cooled to 35°C, resulting in the formation of an emulsified composition. This emulsified composition gave a rich feeling upon application and was excellent in sense of use.

### [Table 7]

**(Table 7)**

| | Component | Blending amount (mass%) | | | |
|---|---|---|---|---|---|
| | | Formulation Example 1 | Formulation Example 2 | Formulation Example 3 | Formulation Example 4 |
| Oil phase 1 | STL C14 | 0.1 | 0.1 | 0.1 | 0.1 |
| | Cetearyl alcohol | 3 | 3 | 3 | 3 |
| | Ceramide NG | 0.01 | 0.01 | 0.01 | 0.01 |
| | Ceramide NP | 0.01 | 0.01 | 0.01 | 0.01 |
| | Ceramide AP | 0.01 | 0.01 | 0.01 | 0.01 |
| Oil phase 2 | Squalane | 50 | 50 | 50 | 50 |
| | Vaseline | 0.5 | 0.5 | 0.5 | 0.5 |
| | Dipentaerythrityl hexa(hydroxystearic acid/stearic acid/rosin acid) | 0.2 | 0.2 | 0.2 | 0.2 |
| | Ethylhexyl methoxycinnamate | 1 | 1 | 1 | 1 |
| | Ethylhexylglycerin | 0.3 | 0.3 | 0.3 | 0.3 |
| | Na hydroxide | Appropriate amount | Appropriate amount | Appropriate amount | Appropriate amount |
| | Xanthan gum | 0.2 | 0.2 | 0.2 | 0.2 |
| | BG | 10 | 10 | 10 | 10 |
| | Glycerin | 5 | 5 | 5 | 5 |
| Aqueous phase | Lecithin | 0.3 | | | |
| | Polysorbate 60 | | 0.3 | | |
| | Sucrose stearate | | | 0.3 | |
| | Na stearoyl glutamate | 0.1 | 0.1 | 0.1 | 0.1 |
| | Polyglyceryl laurate-10 | | | | 0.3 |
| | Water | Remaining amount | Remaining amount | Remaining amount | Remaining amount |
| Additive 1 | Phenoxyethanol | 0.3 | 0.3 | 0.3 | 0.3 |
| Additive 2 | Hydrolyzed collagen | 0.05 | 0.05 | 0.05 | 0.05 |
| | Na hyaluronate | 0.01 | 0.01 | 0.01 | 0.01 |
| | Water | 5 | 5 | 5 | 5 |

### Industrial Applicability

The activator containing the anionic biosurfactant according to an embodiment of the present invention has excellent cell activating properties, low skin irritation, and good foaming durability. Thus, the activator containing the anionic biosurfactant according to an embodiment of the present invention can be widely used not only as an activator but also for cosmetics, quasi-drugs, and the like. The anionic biosurfactant according to an embodiment of the present invention can also be suitably used as various skin condition improving agents or α-gel-containing compositions.

## Claims

1. An activator comprising an anionic biosurfactant.

2. The activator according to claim 1, wherein the anionic biosurfactant includes a biosurfactant having a glycolipid structure.

3. The activator according to claim 1, wherein the anionic biosurfactant includes a succinoyl trehalose lipid.

4. The activator according to claim 1, wherein a content of the anionic biosurfactant is 95 parts by mass or more with respect to 100 parts by mass of the activator.

5. The activator according to claim 1, wherein the activator is an epidermal keratinocyte activator.

6. A cosmetic or a quasi-drug comprising the activator described in any one of claims 1 to 5.

7. A skin condition improving agent comprising an anionic biosurfactant.

8. The skin condition improving agent according to claim 7, wherein the skin condition improving agent is a skin barrier property improving agent.

9. The skin condition improving agent according to claim 7, wherein the skin condition improving agent is a skin moisture retention improving agent.

10. The skin condition improving agent according to claim 7, wherein the skin condition improving agent is a wrinkle formation inhibitory agent.

11. The skin condition improving agent according to claim 7, wherein the skin condition improving agent is a wrinkle improving agent.

12. The skin condition improving agent according to claim 7, wherein the skin condition improving agent is an oxidative stress inhibitory agent.

13. The skin condition improving agent according to claim 7, wherein the skin condition improving agent is a stress inhibitory agent.

14. The skin condition improving agent according to claim 7, wherein the skin condition improving agent is a skin bacterial flora improving agent.

15. An α-gel-containing composition comprising:
an anionic biosurfactant; and
a higher alcohol.
